(19) Europäisches Patentamt
European Patent Office
Office européen des brevets

(11) EP 2 935 252 B1

# (12) EUROPEAN PATENT SPECIFICATION

(45) Date of publication and mention of the grant of the patent:
**05.04.2017 Bulletin 2017/14**

(21) Application number: **13818506.1**

(22) Date of filing: **20.12.2013**

(51) Int Cl.:
***C07D 407/12*** *(2006.01)* ***A61K 31/34*** *(2006.01)*
***A61P 11/06*** *(2006.01)* ***A61P 37/08*** *(2006.01)*

(86) International application number:
**PCT/NL2013/050930**

(87) International publication number:
**WO 2014/098597 (26.06.2014 Gazette 2014/26)**

## (54) NOVEL BIS-CHROMONE DERIVATIVES, METHODS FOR THEIR PREPARATION AND USES THEREOF

NEUARTIGE BIS-CHROMONDERIVATE, HERSTELLUNGS- UND VERWENDUNGSVERFAHREN DAFÜR

NOUVEAUX DÉRIVÉS BIS-CHROMONE, PROCÉDÉS POUR LEUR PRÉPARATION ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2012 EP 12198950**
**09.01.2013 US 201361750371 P**

(43) Date of publication of application:
**28.10.2015 Bulletin 2015/44**

(73) Proprietor: **Rijksuniversiteit te Groningen**
**9712 CP Groningen (NL)**

(72) Inventors:
- **VELEMA, Willem Arend**
  **NL-9747 AG Groningen (NL)**
- **FERINGA, Bernard Lucas**
  **NL-9747 AG Groningen (NL)**
- **SZYMANSKI, Wiktor**
  **NL-9747 AG Groningen (NL)**

(74) Representative: **V.O.**
**P.O. Box 87930**
**2508 DH Den Haag (NL)**

(56) References cited:
**US-A- 3 673 218 US-A- 4 105 781**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The invention relates to new pharmaceutical compounds, methods for their preparation and uses thereof. In particular, it relates to novel bis-chromone derivatives and their therapeutic application, especially in the treatment or prevention of diseases involving mast cell activation, such as allergic diseases.

The prevalence of allergic diseases has increased over the last decades[1,2] and the search for suitable pharmacotherapeutical treatment is ongoing.[3,4] Several options to treat allergic patients are available,[5] including antihistamines, leukotriene inhibitors, corticoids and chromones. This last class of drugs has lost popularity, mainly due to their less pronounced effect and short duration of action.[6] However, chromones show few side-effects,[6] as opposed to the commonly used corticoids,[7,8] which renders them continuously interesting therapeutic agents. Furthermore, they seem to have an interesting, yet not fully understood, mechanism of action.[9]

Chromone (or 1,4-benzopyrone) is a derivative of benzopyran with a keto group on the pyran ring. It is an isomer of coumarin. Derivatives of chromone are collectively known as chromones. Chromones stabilize mast cells,[10] which are effector cells of immediate hypersensitivity reactions and therefore play a key role in allergic diseases.[11] These cells can be found throughout the human body and they secrete granules upon activation.[12,13] The granules contain inflammatory mediators, which induce the symptoms of an allergic reaction, like edema, warmth and itchiness.[12] Activation of mast cells occurs as a result of cross-linking of high affinity IgE receptors on the cell surface by allergens.[14] Chromones bind to the Cromolyn Binding Protein (CBP) on the surface of mast cells and inhibit this activation process.[15] While the exact mechanism of their action is yet to be elucidated, it is hypothesized that chromones interfere with the calcium influx which is necessary for degranulation.[16,17]

Cromoglicic acid (INN) (also referred to as cromolyn (USAN), cromoglycate (former BAN), or cromoglicate) is described as a mast cell stabilizer, and is commonly marketed as the DiSodiumCromoGlycate (DSCG) [18]. DSCG inhibits mast-cell activation and its structure-activity relationships are well studied.[19] The drug consists of two chromone moieties linked by a spacer (Fig. 1A). Altering the length of the spacer changes the inhibitory activity of the drug.[19] See also US 3,419,578. Further reported therapeutic uses of DSCG include the treatment of pathological mineral resorptive states, such as osteoporosis (US 4,501,754)

Recognizing the advantages of chromone-based drugs as well as the need for new and more effective anti-allergic agents, the present inventors aimed at providing a new class of chromone derivatives which have higher potency than the currently available chromone-based drugs. Furthermore, they sought to develop mast cell activation inhibitors that allow for a high level of spatiotemporal control over events of the molecular level. This would facilitate studying mechanisms involved in allergic reactions in a novel manner.

It was surprisingly found that at least some of these goals could be met by the provision of a bis-chromone compound wherein the chromone groups are linked by a spacer containing an azo-moiety. In mast cell degranulation assays, these azo-chromone compounds possess a significantly greater potency than the standard chromone-based anti-allergic agent DSCG.

Accordingly, the invention provides a compound of the general formula I

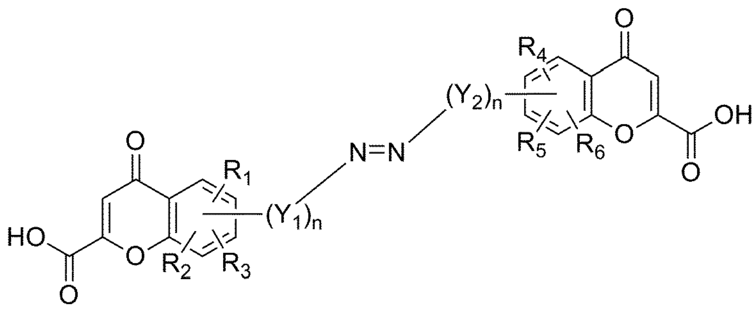

Formula I

or a pharmaceutically acceptable salt, ester or amide thereof, wherein

n is 0 or 1;

$Y_1$ is $X_1$-$Ar_1$ or O-$X_1$-$Ar_1$; and $Y_2$ is $X_2$-$Ar_2$ or O-$X_2$-$Ar_2$, wherein $Ar_1$ and $Ar_2$ are linked to N=N;

wherein $X_1$ and $X_2$ are the same or different and each is a substituted or unsubstituted straight hydrocarbon

chain of 1 to 3 carbon atoms;

wherein $Ar_1$ and $Ar_2$ are the same or different and each is a 5- or 6-membered aromatic or heteroaromatic ring, optionally substituted with one or more substituents selected from the group consisting of halogen, hydroxyl, substituted or unsubstituted $C_1$-$C_4$ alkyl, substituted or unsubstituted $C_1$-$C_4$ lower alkoxy; and

$R_1$, $R_2$, $R_3$, $R_4$, $R_5$ and $R_6$ are the same or different and each is selected from the group consisting of H, halogen, nitro, amino, cyano, hydroxyl, substituted or unsubstituted $C_1$-$C_4$ alkyl, substituted or unsubstituted $C_1$-$C_4$ alkoxy.

Azo-linked bis-chromonyl compounds according to Formula I are not known or suggested in the art. US 3,419,578 discloses compounds wherein the chromone groups are connected via the linker -O-X-O-, wherein X is a saturated or unsaturated, substituted or unsubstituted, straight or branched polymethylene chain. The chain may be interrupted by one or

more carbocyclic rings or oxygen containing heterocyclic rings, oxygen atoms or carbonyl groups.

US 3,673,218 discloses bis-chromonyl compounds wherein the linker is a carbon to carbon bond or a single atom through which the chromone residues are linked. Exemplary single atoms include oxygen, sulphur, or substituted sulphur or nitrogen.

WO2010088455 discloses cromolyn analogs useful as imaging agents for detecting atherosclerotic plaques and for treating atherosclerosis and Alzheimer's Disease. Like in US 3,419,578, the linker is based on a polymethylene chain.

US 4,105,781 relates to chromone compounds wherein the linker is hydroxyl (lower) alkylene, preferably having 1 to 4 carbon atoms.

In one embodiment, the invention provides a bis-chromonyl compound according to Formula I wherein n is 0, such that the azo-linker is directly bound to the chromone-moieties.

In another embodiment, the invention provides a bis-chromonyl compound according to Formula I wherein n is 1, such that the linker comprises an azo-group flanked on each side by an aromatic moiety. Bis-chromonyl compounds comprising an aromatic azo-linker were found to be very potent mast cell stabilizers. The aromatic moieties $Ar_1$ and $Ar_2$ are bound directly to the N=N, and bound to the chromonyls via a lower alkyl ($X_1$; $X_2$) or lower alkoxy spacer (O-$X_1$; O-$X_2$) consisting of 1 to 3 carbon atoms. Thus, $Y_1$ is $X_1$-$Ar_1$ or O-$X_1$-$Ar_1$ and $Y_2$ is $X_2$-$Ar_2$ or O-$X_2$-$Ar_2$, wherein $X_1$ and $X_2$ are the same or different and each is a straight hydrocarbon chain of 1 to 3 carbon atoms. For example, at least one of $X_1$ and $X_2$ is $-CH_2-$ or$-CH_2-CH_2-$. Preferably, $X_1$ and $X_2$ are the same, more preferably each of $X_1$ and $X_2$ is $-CH_2-$.

In one embodiment, at least one of the aromatic moieties is bound to the chromonyl via a lower alkoxy spacer, i.e. $Y_1$ is O-$X_1$-$Ar_1$ and/or $Y_2$ is O-$X_2$-$Ar_2$. For example, $Y_1$ is O-$CH_2$-$Ar_1$ and/or $Y_2$ is O-$CH_2$-$Ar_2$. As another example, $Y_1$ is O-$CH_2$-$CH_2$-$Ar_1$ and/or $Y_2$ is O-$CH_2$-$CH_2$-$Ar_2$.

In another embodiment, at least one of the aromatic moieties is bound to the chromonyl via a lower alkyl spacer, i.e. $Y_1$ is $X_1$-$Ar_1$ and/or $Y_2$ is $X_2$-$Ar_2$. For example, $Y_1$ is $-CH_2$-$Ar_1$ and/or $Y_2$ is $-CH_2$-$Ar_2$. As another example, $Y_1$ is $-CH_2$-$CH_2$-$Ar_1$ and/or $Y_2$ is $-CH_2$-$CH_2$-$Ar_2$. In a specific aspect, both aromatic moieties $Ar_1$ and $Ar_2$ are bound to the respective chromonyls via a lower alkyl spacer. As will be understood, in case of an alkoxy spacer the ether bond is connected to the chromonyls and the X moieties to the aromatic moieties.

Preferably, both aromatic moieties $Ar_1$ and $Ar_2$ are bound to the chromonyl via a lower alkoxy spacer. Preferred compounds include

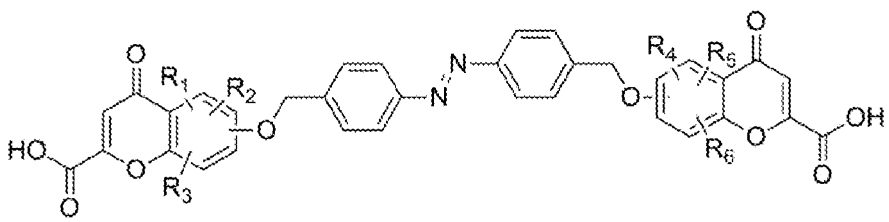

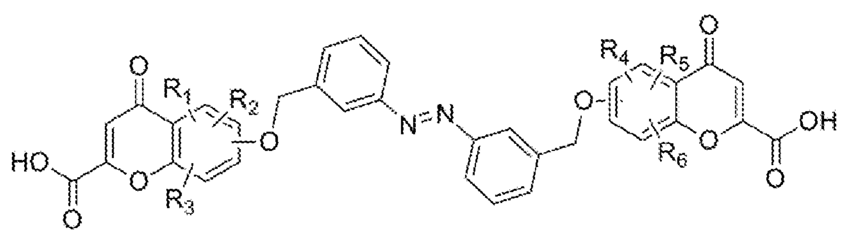

In a compound of the invention, the aromatic moiety is a 5- or 6-membered aromatic ring or heteroaromatic ring with one or two heteroatoms selected from the group of O, N and S. For example, $Ar_1$ and $Ar_2$ can be independently selected from the group consisting of benzene, pyridine, pyrrole, furan, thiophene, pyrazole, imidazole, isoxazole, oxazole, isothiazole, thiazole, pyridazine, pyrimidine and pyrazine. $Ar_1$ and $Ar_2$ may be the same or different. Preferably, at least one of $Ar_1$ and $Ar_2$ is benzene, pyridine, pyrrole, furan or thiophene. Preferably, $Ar_1$ and $Ar_2$ are the same. Most preferably, $Ar_1$ and $Ar_2$ are benzene.

The (hetero)aromatic ring may be substituted with one or more substituents selected from the group consisting of halogen (e.g. fluoro, chloro, bromo, or iodo), hydroxyl, (substituted) lower alkyl (preferably alkyl of 1 to 4 carbon atoms), and (substituted) lower alkoxy (preferably alkoxy of 1 to 4 carbon atoms). In one embodiment, the (hetero)aromatic ring is unsubstituted.

Preferred azo-bis-chromonyl compounds comprising a heteroaromatic ring include

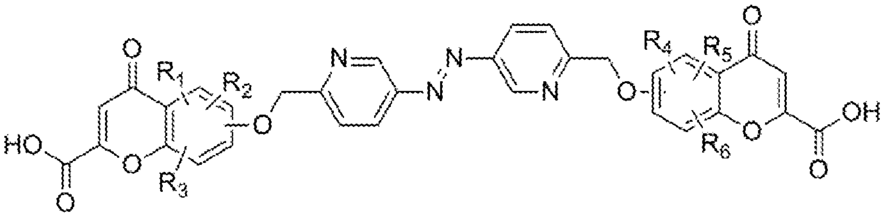

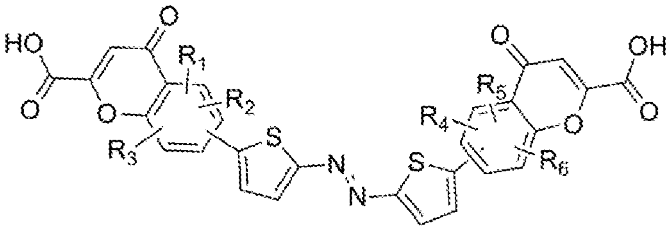

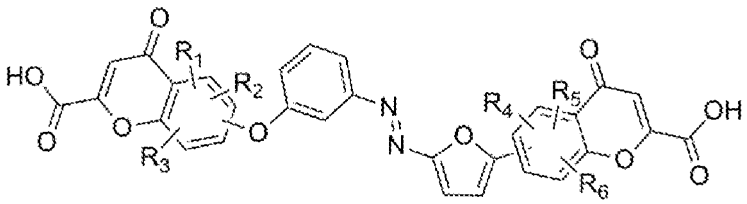

In a compound of the invention, the substituents $R_1$, $R_2$, $R_3$, $R_4$, $R_5$ and $R_6$ on the chromone moieties may be the same or different, and each is selected from the group consisting of H, halogen, nitro, amino, cyano, hydroxyl, (substituted) lower (e.g $C_1$-$C_6$) alkyl and (substituted) lower (e.g. $C_1$-$C_6$) alkoxy. In one embodiment, the substituents on the two chromone moieties of the bis-chromonyl compound are different i.e. $R_1$, $R_2$ and $R_3$ are different from $R_4$, $R_5$ and $R_6$. Preferably, the two chromone moieties of the bis-chromonyl compound are identical i.e. $R_1$, $R_2$ and $R_3$ are the same as $R_4$, $R_5$ and $R_6$. In one embodiment, at least one of the R substituents on each ring is hydrogen, preferably at least two R substituents are hydrogen. Hence, provided are compounds wherein each of $R_2$, $R_3$, $R_5$ and $R_6$ is hydrogen. In a specific aspect, each of $R_1$, $R_2$, $R_3$, $R_4$, $R_5$ and $R_6$ is hydrogen.

The relative positioning of the bond attaching the azo-containing linker to the benzene ring of each of the chromonyl moiety can vary. For example, the azo-linker can be bound to one chromonyl moiety via position a, b, c or d and to the other chromonyl moiety via position to any one of position e, f, g or h in the Formula shown below.

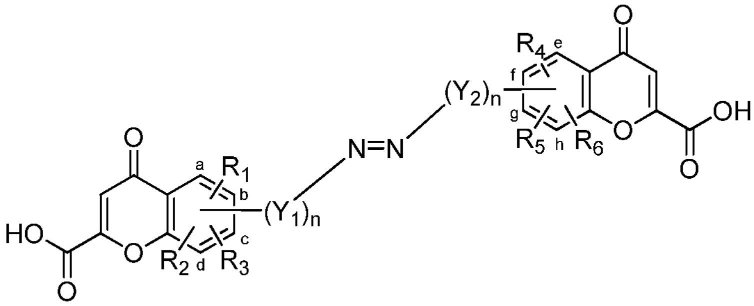

As will be understood, the remaining positions of *a, b, c* and *d* are available for $R_1$, $R_2$ and $R_3$. Likewise, the remaining positions of *e, f, g* and *h* are available for $R_4$, $R_5$ and $R_6$.

Preferably, the azo-linker is bound to one chromonyl moiety via position *a, b* or *c* and to the other chromonyl moiety via position to *e*, *f* or *g.*

In one embodiment, the azo-linker is bound to the respective chromonyl moieties via symmetrical (equivalent) or asymmetrical positions on the benzene ring. Exemplary compounds wherein the azo-linker is connected via asymmetrical positions include compounds E (positions *b* and *e*) and F (positions *d* and *e*). Preferably, the positions are symmetrical. Symmetrical/equivalent positions are position pairs *a* and *e*, *b* and *f, c* and *g* and *d* and *h.* Exemplary compounds wherein the azo-linker is connected via symmetrical positions include compounds A and B (positions *a* and *e*), C and G (positions *b* and *f*) and compound D (positions *c* and *g*). Preferred symmetrical position pairs for azo-linker attachment are *a* and *e*, and *b* and *f.*

In one embodiment, n=1 and the linker is attached to the chromonyl moieties via positions *a* and *e*. See for example compounds A and B, and compounds (E)-5,5'-(((diazene-1,2-diylbis(3,1-phenylene))bis(methylene)) bis(oxy))bis(4-oxo-4H-chromene-2-carboxylate) (MAC) and (E)-5,5'-(((diazene-1,2-diylbis(4,1-phenylene))bis(methylene))bis(oxy)bis(4-oxo-4H-chromene-2-carboxylate) (PAC; see Fig. 1).

In a preferred aspect, the invention provides a compound wherein n=1 and wherein aromatic moieties $Ar_1$ and $Ar_2$ are bound to the chromonyl via a lower alkoxy spacer and wherein $Y_1$ and $Y_2$ are attached to the chromonyl moieties via positions *a* and *e*.

In another embodiment, n=0 and the azo-linker is attached to the chromonyl moieties via positions *b* and *f.* See for example compound (E)-6,6'-(diazene-1,2-diyl)bis(4-oxo-4H-chromene-2-carboxylate)(DAC; see Fig. 1).

Other preferred compounds include (*E*)-5,5'-(((5,5'-(diazene-1,2-diyl)bis(pyridine-5,3-diyl))bis(methylene))bis(oxy))bis(4-oxo-4H-chromene-2-carboxylic acid) (Compound A of Fig. 5), (*E*)-5,5'-(((4,4'-(diazene-1,2-diyl)bis(thiazole-4,2-diyl))bis(methylene))bis(oxy))bis(4-oxo-4H-chromene-2-carboxylic acid) (Compound B of Fig. 5), (*E*)-6-(2-(4-((3-(2-carboxy-4-oxo-4H-chromen-5-yl)phenyl)diazenyl)thiophen-2-yl)ethyl)-4-oxo-4H-chromene-2-carboxylic acid (Compound E of Fig. 5) and (*E*)-6-((5-((2-carboxy-4-oxo-4H-chromen-6-yl)diazenyl)furan-2-yl)oxy)-4-oxo-4H-chromene-2-carboxylic acid (Compound G of Fig. 5).

Pharmaceutically acceptable salts, esters or amides thereof include salts, esters and amides of one or more of the carboxylic acid functions present and esters of any hydroxylic functions present. Salts of the bis-chromonyl compounds include salts with physiologically acceptable cations, for example, ammonium salts, metal salts such as alkali metal salts (e.g. sodium, potassium and lithium salts) and alkaline earth metal salts (e.g. magnesium and calcium salts) and salts with organic bases, e.g. amine salts such as piperidine, triethanolamine and diethylaminoethylamine salts. Esters which may be mentioned include simple alkyl esters (e.g. methyl, ethyl, propyl, isopropyl, butyl and tertiary butyl esters) and amides which include simple amides and more complex amides with amino acids, such as glycine.

The compounds MAC and PAC show an almost 100 times higher potency than the chromone drug DSCG, basing on β-hexosaminidase release assays (Fig. 3). This large increase in potency makes MAC and PAC very interesting anti-allergic agents. Thus, the novel compounds as disclosed herein are a highly attractive alternative to known chromone-based drugs.

In one embodiment, the invention provides a pharmaceutical composition comprising a compound according to the invention and a pharmaceutically active carrier, diluent or excipient. Preferred compounds include MAC, PAC, DAC (see Fig. 1) and compounds A through G shown in Figure 5. Most preferred are compounds A through G wherein each of $R_1$-$R_6$ is hydrogen.

The composition comprises a therapeutically effective dose of the chromone-derivative. The skilled person will be able to determine for each application the therapeutically effective dose, taking into account e.g. the nature and severity of the disease, age and/or weight of the subject to be treated, and the dose regimen. For example, for the treatment of asthma, a dose up to 20 mg (e.g. 1-15 mg) may be administered at 4-12 hour intervals by inhalation.

The pharmaceutical composition can be formulated in any desirable form, depending on the intended use and route of administration. For example, it is formulated as a nasal spray, inhalator, eye drops, oral formulation, nebulizer

solution or topical formulation, like a solution, gel, paste, ointment or cream. Also provided is the use of a compound according to the invention as therapeutic or prophylactic agent.

A pharmaceutical composition may of course contain one or more additional therapeutically active ingredients. In one embodiment, it comprises at least one further ingredient capable of inhibiting the release of mediators from mast cells. The further ingredient(s) may be a novel azochromone compound of the invention and/or a previously described mast cell stabilizer. Examples of other compounds that inhibit the release of mediators from mast cells include olopatadine ((Z)-11-(3-(dimethylamino)propylidene)-6,11-dihydrodibenz(b,e)oxepine-2-acetic acid hydrochloride), amlexanox (C16 H14 N2 04 ; 2-Amino-7-(1-methylethyl)-5-oxo-5H-[1]benzopyrano(2,3-b)pyridine-3-carboxy lic acid), ketotifen (C19 H19 NOS; 4,9-Dihydro-4-(1-methyl-4-piperidin-ylidene)-10H-benzo[4,5]cyclohepta[1,2-b]thiophen-10-one) and ketotifen fumarate (C23 H23 NO5 S), lodoxamide tromethamine (N,N'-(2-chloro-5-cyano-m-phenylene)dioxamic acid tromethamine salt), minocromil (6-Methylamino-4-oxo-10-propyl-4H-pyrano[3,2-g]quinoline-2,8-dicarboxylic acid) and its sodium salt, repirinast (C20 H21 NO5 ;5,6-Dihydro-7,8-dimethyl-4,5-dioxo-4H-pyrano[3,2-c]quinoline-2-carboxylic acid 3-methylbutylester), suplatast tosylate ((+/-)-(2-{[p-(3-Ethoxy-2-hydroxypropoxy)phenyl]carbamoyl}ethyl]dimethyl sulphonium p-toluenesulphonate), tiacrilast ((E)-6-(methylthio)-4-oxo-3(4H)-quinazolineacrylic acid) and its sodium salt, tranilast (C18 H 17 NO5 ; 2 [[3-(3,4-Dimethoxyphenyl)-1-oxo-2-propenyl]amino]benzoic acid), taxanox (C13 H6 ClN5 02 ; 9-Chloro-7-(1H-tetrazol-5-yl)-5H-[1]benzopyrano[2,3-b]pyridin-5-one) and its sodium salt, and zaprinast (1,4-Dihydro-5-(2-propoxyphenyl)-1,2,3-triazolo[4,5-d]pyrimidin-7-one.

Mast cells play a role in immediate hypersensitivity and other inflammatory reactions by releasing a variety of chemical mediators upon activation. Mast cells play a key role in the inflammatory process. Mast cells can be stimulated to degranulate by crosslinking of surface IgE attached to FCepsilonRI, by allergens, chemokines, cytokines, or by activated complement proteins. When activated, a mast cell rapidly releases its characteristic granules and various hormonal mediators into the interstitium. These mediators, some of which are stored inside granules in the cytoplasm, include biogenic amines such as histamine, lipid mediators such as leukotrienes, prostaglandins, and platelet-activating factor, cytokines and enzymes. As exemplified herein below, the azochromonone compounds of the invention are very potent inhibitors of mast cell degranulation invoked by an established promoter of mast cell degranulation. $IC_{50}$ values below 300 $\mu$M and even below 30 $\mu$M were observed under conditions wherein the reference bis-chromonyl compound DSCG did not show any inhibitory activity ($IC_{50}$> 1 mM).

Thus, a further aspect of the invention provides a bis-chromonyl compound for use in a method of treatment or prophylaxis of a disease involving unwanted or excessive mast cell activation, typically resulting in an excessive or undesirable release of mediators from mast cells.

In one embodiment, the disease is an allergic disease. The term "allergic disease" as used herein refers to any hypersensitivity condition of the immune system. Exemplary conditions to be treated with a compound of the invention include asthma, allergic rhinitis, mastocytosis, dermatographic urticaria, eczema, psoriasis, ulcerative colitis, food allergy and vulvar vestibulitis. For example, oral formulations can be used to treat the diarrhea, flushing, headaches, vomiting, urticaria, abdominal pain, nausea, and itching of mastocytosis, which is an accumulation of mast cells in the tissues. Opthalmic and nasal solutions can be used to treat the itching, redness, swelling, sneezing, tearing, and discharge of allergic conjunctivitis and allergic rhinitis. An inhalation aerosol is useful as prophylactic agents in the management of asthma.

An azo-linked bis-chromonyl compound of the invention, which inhibits the release of histamine and other mediators from mast cells that have been sensitized by specific antigens, is preferably used pharmacologically as an anti-asthmatic /anti-allergic.

Mast cells have also been implicated in the pathology associated with the autoimmune disorders rheumatoid arthritis, bullous pemphigoid, and multiple sclerosis. They have been shown to be involved in the recruitment of inflammatory cells to the joints (e.g. rheumatoid arthritis) and skin (e.g. bullous pemphigoid) and this activity is dependent on antibodies and complement components. Hence, symptoms associated with these conditions may also be alleviated upon treatment with a mast cell stabilizing compound as disclosed in the present invention.

In yet another embodiment, the compound is used in a method for treatment of pathological mineral resorptive states. For example, a composition is formulated for treating bone resorption and comprises one or more known anti-osteoporotic agents, e.g. selected from anabolic steroids, various phosphorus-containing agents, vitamin D and related substances, estrogenic steroids, and calcitonin. Also, certain aromatic carboxylic acids have been described as useful anti-osteoporotic agents. For a detailed review and discussion of such anti-osteoporotic agents, see U.S. Pat. Nos. 4,125,621 or 4,101,688.

A further aspect of the invention relates to the use of an azo-linked bis-chromonyl compound for use as *in vitro* mast cell stabilizer. For example, provided is a method for investigating in vitro the mechanism of mast cell activation, comprising contacting a mast cell culture with a mast cell stabilizing azo-containing bis-chromonyl compound as provided herein, wherein said compound is able to switch reversibly between an active state and an inactive state when irradiated with light. The light of a first wavelength to switch the compound from the inactive state to an active state and irradiated with the light of a second wavelength is different to the first wavelength to switch the compound from the active state to

the inactive state. Preferably, the compound is (E)-5,5'-(((diazene-1,2-diylbis(4,1-phenylene))bis(methylene))bis(oxy))bis(4-oxo-4H-chromene-2-carboxylate).

Azo-linked bis-chromonyl compounds of the invention can be synthesized using standard organic chemistry using commercially available starting materials. The skilled person will be able to select the starting compounds and the appropriate synthetic schemes.

In one embodiment, a method for preparing a compound wherein n=0 comprises the steps of (i) providing chromone ethyl ester from hydroaxyacetophenone and diethyloxalate (ii) converting chromone ethyl ester into nitroso chromone via sequential nitration, reduction and oxidation; (iii) azobenzene formation from nitroso chromone and amino chromone; and (iv) saponification. See for example Scheme 1 herein below.

In another embodiment, the invention provides a method for preparing a compound according to Formula I wherein n=1 comprising the steps of (i) reacting m/p-toluidine and m/p-nitroso toluene to afford methylated azobenzene (ii) subjecting methylated azobenzene to radical bromination (iii) introducing -5-hydroxychromone methyl ester to both methylene groups and (iv) saponification. See for example Scheme 2 herein below.

LEGEND TO THE FIGURES

**Figure 1: Molecular structures of inhibitors.** (A) Molecular structure of the prior art mast cell activation inhibitor DiSodiumCromoglycate (DSCG) and the three exemplary novel AzoChromone compounds MAC, PAC and DAC. (B) Principle of photoswitchable mast cell activation inhibitors Envisioned mode of action of MAC and PAC. When two chromone groups (illustrated as spheres) of the inhibitor in one of the photoisomeric forms are bound to the cromolyn binding protein (CBP) mast cell activation is inhibited. When the molecule photoisomerizes to the other form, only one chromone group can bind and mast cell activation is not inhibited.

**Figure 2: Photochemical properties of novel AzoChromone compounds DAC, MAC and PAC.** (A-C) UV-Vis absorption spectra of DAC (125 μM), MAC (128 μM) and PAC (100 μM) in water. The black lines represent the spectra of the non-irradiated forms and the grey lines of the 365 nm irradiated forms. (D-F) Reversible photochromism of photoswitchable inhibitors.

**Figure 3: Concentration response curves of mast cell activation inhibitors** (n=3). Degranulation was evoked by 1.0 μM of Compound 48/80. The novel mast cell activation inhibitors MAC and PAC show inhibitory activity in the same concentration range with $IC_{50}$ values of 10 μM and 28 μM respectively. DAC shows to be a less potent inhibitor with an $IC_{50}$ value of 272 μM, but is still more potent than the original drug DSCG, which did not show any inhibitory activity under these conditions ($IC_{50}$>1 mM).

**Figure 4: Inhibitory activity of the non-irradiated and 365 nm light-irradiated forms PAC.** A difference in activity was observed between the two forms of PAC. Non-irradiated PAC was 25% more active than irradiated PAC at a concentration of 10 μM.

**Figure 5: Exemplary Azo-chromone compounds of the invention.**

EXPERIMENTAL SECTION

The invention is exemplified by the design and synthesis of three analogs of DSCG, with an azobenzene photoswitch incorporated in their structure. The inhibitory capacity of the molecules, named DAC, MAC and PAC (Figure 1A), in both isomeric forms was tested on human mast cell cultures. Remarkably, the molecules showed to have a considerably higher inhibitory effect on an evoked degranulation in mast cell cultures as compared to DSCG. Furthermore, PAC showed a difference in inhibitory capacity in two isomeric forms. It is anticipated that external control of mast cell activation can be achieved by switching between the two forms with light.

## Materials and Methods

**LAD2 Cell culture** The human mast cell line LAD2 was kindly provided by Dr. A. Kirshenbaum (National Institute of Allergy and Infectious Diseases, Bethesda, MD). LAD2 cells were cultured in suspension at 37 °C, 95% relative humidity and 5% $CO_2$ in StemPro-34 Serum-free medium (Invitrogen) supplemented with 2 mM L-glutamine (Sigma Aldrich), 100 IU/mL penicillin (Sigma Aldrich), 100 μg/mL streptomycin (Sigma Aldrich) and 100 ng/ml rhSCF (Invitrogen). Cells were not allowed to grow beyond a density of 5.0 x $10^5$ cells/mL and fresh medium was supplied once a week.

β-**Hexosaminidase Assay** β-Hexosaminidase release was measured according to a previously published method.[28] In short, 50 μL of supernatant was transferred to a 96-well plate. To this 100 μL of a 2 mg/mL solution of p-nitrophenyl- N-acetyl-D-glucosaminidine in citrate buffer pH 4.5 was added. The plate was incubated at 37 °C for 1 h. The reaction was stopped by adding 100 μL of 0.4 M glycine solution and the absorbance was measured on a plate reader at a wavelength of 405 nm. Subsequently, the percentage released β-hexosaminidase was determined by comparison with the absorption of cell lysate of the same cells using the following formula:

$$\frac{supernatant}{supernatant+lysate}\, x\ 100\% = \%\ Released\ \beta - Hexosaminidase.$$

**Inhibitory Activity Tests** LAD2 cells were suspended in HEPES buffer at a concentration of 50,000 cells/mL. 80 μL of cell suspension was transferred to a microreaction cup to which 10 μL of inhibitor solution was added. This was incubated at 37 °C for 30 min after which 10 μL of a Compound 48/80 solution was added. Next, the cell suspension was incubated for 30 min at 37 °C and the *β*-hexosaminidase release was assayed.

**Photoswitching Experiments** Irradiation experiments were performed with a Spectroline ENB-280C/FE UV lamp (365 nm) and Thor Labs OSL1-EC Fiber Illuminator (white light).

**Synthesis.** *General.* For synthesis all chemicals were obtained from commercial sources and used as received unless stated otherwise. Solvents were reagent grade. Thin-layer chromatography (TLC) was performed using commercial Kieselgel 60, F254 silica gel plates. Flash chromatography was performed on silica gel (Silicycle Siliaflash P60, 40-63 m, 230-400 mesh). Drying of solutions was performed with $MgSO_4$ and solvents were removed with a rotary evaporator. Chemical shifts for NMR measurements were determined relative to the residual solvent peaks. The following abbreviations are used to indicate signal multiplicity: s, singlet; d, doublet; t, triplet; q, quartet; m, multiplet; brs, broad signal; appt, apparent triplet. HRMS (ESI) spectra were obtained on a Thermo scientific LTQ Orbitrap XL. Melting points were recorded using a Buchi melting point B-545 apparatus. UV/Vis absorption spectra were recorded on an Agilent 8453 UV-Visible Spectrophotometer using Uvasol grade solvents. All compounds whose $IC_{50}$ values were determined had purities ≥ 95%. Purities were determined with analytical RP-HPLC performed on a JASCO PU-980 chromatography system using a GRACE Alltima HP C18 5μ column. Compounds were detected with a JASCO UV-1575 UV-Vis intelligent detector.

*Ethyl 4-oxo-4H-chromene-2-carboxylate* **(1)** 2-Hydroxyacetophenone (2.04 g, 15 mmol) and diethyl oxalate (5.12 g, 35 mmol) were dissolved in ethanol (10 mL) and added to a solution of sodium (1.49 g, 65 mmol) in ethanol (100 mL). The reaction mixture was heated at reflux for 1 h, after which it was cooled down and acidified with concentrated HCl until a white precipitate was formed. The precipitate was filtered off and the filtrate was concentrated and extracted with ethyl acetate, washed with brine and dried ($MgSO_4$) After evaporation a solid was obtained. This was recrystallized from methanol/diisopropylether (4:1) to yield 2.45 g (75%) of a white solid. Mp. 66-68°C.

$^{1}$H-NMR (400 MHz, $CDCl_3$): δ 8.21 (dd, J = 7.9, 1.7 Hz, 1H), 7.75 (ddd, J = 8.7, 7.1, 1.7 Hz, 1H), 7.62 (dd, J = 8.6, 1.0 Hz, 1H), 7.46 (ddd, J = 8.1, 7.1, 1.1 Hz, 1H), 7.12 (s, 1H), 4.47 (q, J = 7.1 Hz, 4H), 1.41 (t, J = 7.2 Hz, 3H). $^{1}$H-NMR spectrum in agreement with published data.[22]

*Ethyl 6-nitro-4-oxo-4H-chromene-2-carboxylate* **(2)** Compound **1** (200 mg, 0.92 mmol) was suspended in 65% nitric acid (0.12 mL) and the mixture was cooled on ice. Concentrated sulfuric acid (1.5 mL) was added and the mixture was stirred for 2 h at room temperature, poured on ice-cooled water and the formed precipitate was filtered off to yield 195 mg (81%) of a white solid. Mp. 178-179 °C. $^{1}$H-NMR (400 MHz, $CDCl_3$): δ 9.07 (d, J = 2.8 Hz, 1H), 8.57 (dd, J = 9.2, 2.8 Hz, 1H), 7.78 (d, J = 9.2 Hz, 1H), 7.19 (s, 1H), 4.50 (q, J = 7.2 Hz, 2H), 1.46 (t, J = 7.1 Hz, 3H). $^{13}$C-NMR (100 MHz, $CDCl_3$): δ 176.8, 159.7, 158.7, 152.8, 145.2, 128.9, 124.4, 122.5, 120.6, 115.0, 63.5, 14.1. HR-MS (ESI, $[M+H]^+$): Calcd. for $C_{12}H_{10}NO_6$: 264.0508; Found: 264.0502.

*Ethyl 6-amino-4-oxo-4H-chromene-2-carboxylate* **(3)** Compound **2** (300 mg, 1.14 mmol) was dissolved in benzene (100 mL) and stirred overnight with 10% palladium on charcoal (15 mg) under a hydrogen atmosphere (balloon) at room temperature. The mixture was filtered over celite. The filtrate was evaporated yielding 300 mg of orange needles. Mp. 177-178 °C. $^{1}$H-NMR (400 MHz, $CDCl_3$): δ 7.45 (d, J = 9.0 Hz, 1H), 7.35 (d, J = 2.9 Hz, 1H), 7.11- 7.03 (m, 2H), 4.45 (q, J = 7.1 Hz, 2H), 3.92 (brs, 2H), 1.43 (t, J = 7.1 Hz, 3H). $^{13}$C-NMR (100 MHz, $CDCl_3$): δ 178.4, 160.8, 151.7, 149.6, 144.6, 125.4, 123.2, 119.8, 113.6, 107.4, 62.8, 14.1. HR-MS (ESI, $[M+H]^+$): Calcd. for $C_{12}H_{12}NO_4$: 234.0766; Found: 234.0762.

*Ethyl 6-nitroso-4-oxo-4H-chromene-2-carboxylate* **(4)** Compound **3** (100 mg, 0.43 mmol) was dissolved in DCM (2 mL), a solution of oxone (422 mg, 0.69 mmol) in $H_2O$ (8 mL) was added to this and the biphasic mixture was stirred at room temperature for 2 h. The organic layer was separated and the aqueous layer was extracted twice with DCM. The combined organic layers were washed with 1 M aq. HCl, saturated $NaHCO_3$ and brine and dried ($MgSO_4$). Evaporation of the solvent yielded 75 mg (70%) of a green solid. Mp. 144-145 °C. $^{1}$H-NMR (400 MHz, $CDCl_3$): δ 9.31 (d, J = 2.1 Hz, 1H), 7.77 - 7.65 (m, 2H), 7.21 (s, 1H), 4.50 (q, J = 7.1 Hz, 2H), 1.46 (t, J = 7.1 Hz, 3H). $^{13}$C-NMR (100 MHz,

$CDCl_3$): δ 177.9, 161.6, 159.9, 159.2, 152.6, 125.6, 124.9, 120.8, 120.2, 115.0, 63.4, 14.1. HR-MS (ESI, $[M+H]^+$): Calcd. for $C_{12}H_{10}NO_5$: 248.0559; Found: 248.0552.

*Diethyl 6,6'-(diazene-1,2-diyl)bis(4-oxo-4H-chromene-2-carboxylate)* **(5)** Compounds **3** (70.8 mg, 0.304 mmol) and **4** (75.0 mg, 0.304 mmol) were dissolved in glacial acetic acid (2.5 mL) and stirred for 2 d. The solution was diluted with water and extracted with DCM. The organic phase was washed with water (4x) and brine and dried ($MgSO_4$). Recrystallization from DCM yielded 60 mg (43%) of a light orange solid. Mp. 254-255 °C (dec.). $^1H$-NMR (400 MHz, $CDCl_3$): δ 8.79 (d, J = 2.4 Hz, 2H), 8.35 (dd, J = 9.0, 2.4 Hz, 2H), 7.77 (d, J = 9.0 Hz, 2H), 7.18 (s, 2H), 4.50 (q, J = 7.2 Hz, 4H), 1.46 (t, J = 7.1 Hz, 6H). $^{13}C$-NMR (100 MHz, $CDCl_3$): δ 178.1, 160.3, 157.5, 152.4, 149.4, 127.2, 125.0, 122.8, 120.2, 114.9, 63.2, 14.1. HR-MS (ESI, $[M+H]^+$): Calcd. for $C_{25}H_{18}N_2O_8$: 463.1136 ; Found: 463.1131.

*Sodium (E)-6,6'-(diazene-1,2-diyl)bis(4-oxo-4H-chromene-2-carboxylate)* **(6)** (**DAC**) To a solution of compound **5** (50 mg, 0.11 mmol) in ethanol (2 mL) was added aq. NaOH (2.5 M, 0.128 mL) dropwise at 0 °C. The reaction mixture was heated at reflux for 2 h after which the insoluble product was filtered off and purified by recrystallization from methanol yielding 45 mg (91%) of a red solid. $^1H$-NMR (400 MHz, Methanol-d4): δ 8.71 (d, J = 2.4 Hz, 2H), 8.43 (dd, J = 9.0, 2.5 Hz, 2H), 7.90 (d, J = 9.0 Hz, 2H), 7.04 (s, 2H). $^{13}C$-NMR (100 MHz, Methanol-d4): δ 180.2, 164.2, 160.3, 157.9, 149.3, 126.7, 124.2, 121.5, 120.3, 111.1.HR-MS (ESI, $[M+Na]^+$): Calcd. for $C_{20}H_9N_2O_8Na$ : 451.0154 ; Found: 451.0149.

*Methyl 5-hydroxy-4-oxo-4H-chromene-2-carboxylate* **(7)** 2,6-Dihydroxyacetophenone (1.50 g, 9.90 mmol) and dimethyloxalate (5.80 g, 49.3 mmol) were dissolved in 0.5 M MeONa/MeOH (100 mL) and heated at reflux overnight. The solvent was removed in vacuo and the slurry was dissolved in water (100 mL) and subsequently acidified with concentrated HCl. The precipitate was filtered off and dissolved in MeOH (50 mL) and concentrated HCl (10 mL). This solution was heated at reflux for 2 h after which the crude product was purified by flash chromatography (Silicagel, 40-63 μm, pentane/AcOEt, 9:1, v/v) yielding 762 mg (33%) of a yellow powder. $^1H$-NMR (400 MHz, $CDCl_3$): δ 7.61 (appt, J=8.4 Hz, 1H), 7.03-7.06 (m, 2H), 6.85 (d, J=8.3 Hz, 1H), 4.02 (s, 3H). $^1H$-NMR spectrum in agreement with published data.[24]

*1-Methyl-3-nitrosobenzene* **(8a)** 3-Methylaniline (708 mg, 6.61 mmol) was dissolved in DCM (20 mL), a solution of oxone (8.10g, 13.2 mmol) in water (80 mL) was added to this and the resulting biphasic mixture was stirred at room temperature for 30 min. The organic layer was separated and the aqueous layer was extracted twice with DCM. The combined organic layers were washed with 1 M aq. HCl, saturated $NaHCO_3$ and brine and dried ($MgSO_4$). The crude product was purified by flash chromatography (Silicagel, 40-63 μm, pentane/AcOEt, 4:1, v/v), yielding 430 mg (54%) of a light green solid. $^1H$-NMR (400 MHz, $CDCl_3$): δ 7.77 (d, J=6.4 Hz, 1H), 7.63 (s, 1H), 7.48-7.54 (m, 2H), 2.50 (s, 3H). $^1H$-NMR spectrum in agreement with published data.[32]

*1-Methyl-4-nitrosobenzene* **(8b)** 4-Methylaniline (5.00 g, 33.1 mmol) was dissolved in DCM (100 mL), a solution of oxone (40.7 g, 66.2 mmol) in water (400 mL) was added to this and the resulting biphasic mixture was stirred at room temperature for 30 min. The organic layer was separated and the aqueous layer was extracted twice with DCM. The combined organic layers were washed with 1 M aq. HCl, saturated $NaHCO_3$ and brine and dried ($MgSO_4$) Evaporation of the solvent yielded 2.34 g (59%) of a light green solid. $^1H$-NMR (400 MHz, $CDCl_3$): δ 7.81 (d, J=8.2 Hz, 2H), 7.39 (d, J=8.0 Hz, 2H), 2.44 (s, 3H). $^1H$-NMR spectrum in agreement with published data.[33]

*3, 3'-Dimethylazobenzene* **(9a)** Compound **8a** (500 mg, 4.13 mmol) and 3-methylaniline (369 mg, 3.44 mmol) were dissolved in glacial acetic acid (33 mL) and the mixture was stirred overnight. The solution was diluted with water and extracted with ethyl acetate. The organic phase was washed four times with water and once with brine and dried ($MgSO_4$). The crude product was filtered through silica yielding 400 mg (55%) of an orange solid. $^1H$-NMR (400 MHz, $CDCl_3$): δ 7.70-7.74 (m, 4H), 7.41 (appt, J=8.0 Hz, 2H), 7.29 (d, J=7.6 Hz, 2H), 2.46 (s, 6H). $^1H$-NMR spectrum in agreement with published data.[34]

*4,4'-Dimethylazobenzene* **(9b)** Compound **8b** (300 mg, 2.48 mmol) and 4-methylaniline (292 mg, 2.73 mmol) were dissolved in glacial acetic acid (20 mL) and stirred overnight. The solution was diluted with water and extracted with ethyl acetate. The organic phase was washed four times with water and once with brine and dried ($MgSO_4$). The crude product was purified by flash chromatography (Silicagel, 40-63 μm, pentane/$Et_2O$, 9:1, v/v) yielding 450 mg (86%) of an orange solid. $^1H$-NMR (400 MHz, $CDCl_3$): δ 7.81 (d, J=8.4 Hz, 4H), 7.30 (d, J=8.4 Hz, 4H), 2.43 (s, 6H). $^1H$-NMR spectrum in agreement with published data.[35]

*3,3'-Bis(bromomethyl)azobenzene* **(10a)** To a solution of compound **9a** (1.30 g, 6.18 mmol) in 60 mL of $CCl_4$ was added NBS (2.50 g, 14.2 mmol) and AIBN (80 mg, 0.48 mmol). The resultant solution was stirred overnight at 70°C, then filtered and the filtrate was washed with hot water and brine and dried ($MgSO_4$). After evaporation the product was recrystallized from acetonitrile yielding 800 mg (35%) of an orange solid. Mp. 140-141 °C. $^1H$-NMR (400 MHz, $CDCl_3$): δ 7.95 (m, 2H), 7.87 (m, 2H), 7.52 (m, 4H), 4.59 (s, 4H). $^1H$-NMR spectrum in agreement with published data.[34]

*4,4'-Bis(bromomethyl)azobenzene* **(10b)** To a solution of compound **9b** (300 mg, 1.43 mmol) in 20 mL of $CCl_4$ was added NBS (584 mg, 3.30 mmol) and AIBN (18 mg, 0.10 mmol). The resultant solution was stirred overnight at 70 °C, then filtered and the filtrate was washed with hot water and brine and dried ($MgSO_4$). After evaporation of the solvent, the product was recrystallized from acetonitrile yielding 220 mg (42%) of an orange solid. Mp. 183-185 °C. $^1H$-NMR (400 MHz, $CDCl_3$): δ 7.89 (d, J=8.4 Hz, 4H), 7.54 (d, J=8.4 Hz, 4H), 4.56 (s, 4H). $^1H$-NMR spectrum in agreement with published data.[35]

*(E)-dimethyl5, 5'-(((diazene-1,2-diylbis(3,1-phenylene))bis(methylene))bis(oxy))bis(4-oxo-4H-chromene-2-carboxylate)* **(11a)** To a solution of compounds **7** (638 mg, 2.73 mmol) and **10a** (400 mg, 1.09 mmol) in 80 mL acetonitrile was added $K_2CO_3$ (451.93 mg,3.27 mmol) and the resulting mixture was stirred overnight at 65 °C. The solution was concentrated in vacuo and the crude product was purified using flash chromatography (Silicagel, 40-63 μm, DCM/Methanol, 95:5, v/v) yielding 520 mg (70%) of a light orange solid. $^1$H-NMR (400 MHz, $CDCl_3$): δ 8,06 (s, 2H), 7.90-7.85 (m, 4H), 7.56-7.61 (m, 4H), 7.18 (d, J = 8.5 Hz, 2H), 7.02 (s, 2H), 6.91 (d, J = 8.3 Hz, 2H), 5.38 (s, 4H), 4.00 (s, 6H). $^{13}$C NMR (100 MHz, $CDCl_3$): δ 177.6, 161.1, 158.4, 158.0, 152.7, 150.1, 137.5, 134.7, 129.7, 129.4, 122.6, 120.7, 116.7, 113.8, 111.1, 108.9, 70.6, 53.4. HR-MS (ESI, $[M+H]^+$): Calcd. for $C_{36}H_{27}N_2O_{10}$: 647.1660 ; Found: 647.1603.

*(E)-dimethyl5, 5'-(((diazene-1,2-diylbis(4,1-phenylene))bis(methylene))bis(oxy))bis(4-oxo-4H-chromene-2-carboxylate)* **(11b)** To a solution of compounds **7** (100 mg, 0.45 mmol) and **10b** (75.8 mg, 0.21 mmol) in acetonitrile (25 mL) was added $Cs_2CO_3$ (201 mg, 0.62 mmol) and the resulting mixture was stirred at 65 °C for 3 h. The solution was concentrated in vacuo and the residue was dissolved in DCM, washed with 1 M aq. HCl, saturated $NaHCO_3$ and brine and dried ($MgSO_4$). After evaporation the product was recrystallized from DCM yielding 27 mg of a light orange solid. Mp. 235-237 °C (dec). $^1$H-NMR (400 MHz, $CDCl_3$): δ 7.96 (d, J = 8.0 Hz, 4H), 7.76 (d, J = 8.1 Hz, 4H), 7.58 (t, J = 8.0 Hz, 2H), 7.18 (d, J = 8.2 Hz, 2H), 7.02 (s, 2H), 6.90 (d, J = 8.3 Hz, 2H), 5.36 (s, 4H), 4.00 (s, 6H). $^{13}$C-NMR (100 MHz, $CDCl_3$): δ 177.7, 161.1, 158.4, 158.0, 152.2, 150.1, 139.3, 134.6, 127.2, 123.2, 116.6, 113.8, 111.1, 108.7, 70.5, 53.4. HR-MS (ESI, $[M+H]^+$): Calcd. for $C_{36}H_{27}N_2O_{10}$: 669.1485 ; Found: 669.1480.

*Sodium (E)-5,5'-(((diazene-1,2-diylbis(3,1-phenylene))bis(methylene))bis(oxy))bis(4-oxo-4H-chromene-2-carboxylate)* **(12a) (MAC)** To a solution of compound **11a** (100 mg, 0.15 mmol) in ethanol (5 mL) was added aq. NaOH (2.5 M, 186 μL) dropwise at 0°C. The reaction mixture was heated at reflux for 2 h after which the insoluble product was filtered off yielding 85 mg (86%) of an orange solid.
$^1$H-NMR (400 MHz, Methanol-$d_4$): δ 8.12 (s, 2H), 7.86 (d, J = 8.0 Hz, 2H), 7.81 (d, J = 8.0 Hz, 2H), 7.63 (m, 4H), 7.26 (d, J = 8.4 Hz, 2H), 7.06 (d, J = 8.4 Hz, 2H), 6.88 (s, 2H), 5.42 (s, 4H). $^{13}$C-NMR (100 MHz, Methanol-$d_4$): δ 180.4, 164.7, 158.3, 158.1, 157.9, 152.7, 138.3, 134.5, 129.1, 121.2, 113.8, 112.5, 110.9, 108.5, 69.9. HR-MS (ESI, $[M+Na]^+$): Calcd. for $C_{34}H_{22}N_2O_{10}Na$: 641.1167 ; Found: 641.1140.

*Sodium (E)-5,5'-(((diazene-1,2-diylbis(4,1-phenylene))bis(methylene))bis(oxy))bis(4-oxo-4H-chromene-2-carboxylate)* (**12b**) (**PAC**) To a solution of compound **11b** (25 mg, 0.04 mmol) in ethanol (3 mL) was added aq. NaOH (2.5 M, 50 μL) dropwise at 0 °C. The reaction mixture was heated at reflux for 2 h after which the insoluble product was filtered off yielding 23 mg (91%) of an orange solid. $^1$H-NMR (400 MHz, Methanol-$d_4$): δ 7.96 (d, J = 8.3 Hz, 4H), 7.80 (d, J = 8.2 Hz, 4H), 7.67 (t, J = 8.4 Hz, 2H), 7.28 (d, J = 8.5 Hz, 2H), 7.05 (d, J = 8.3 Hz, 2H), 6.89 (s, 2H), 5.41 (s, 4H). $^{13}$C-NMR (100 MHz, Methanol-$d_4$): δ 180.3, 164.6, 158.3, 158.1, 158.0, 155.8, 152.1, 140.2, 134.4, 127.3, 122.5, 112.5, 110.9, 108.4, 69.8. HR-MS (ESI, $[M+Na]^+$): Calcd. for $C_{34}H_{22}N_2O_{10}Na$: 641.1167 ; Found: 641.1155.

**Design of photoswitchable inhibitors** The structure of photoswitchable inhibitors DAC, MAC and PAC was based on the known mast cell activation inhibitor DSCG. This drug consists of two chromone groups linked by a spacer (Fig. 1A). The length of the spacer influences the inhibitory activity of the drug.[19] We chose an azobenzene unit as the photoswitch, since the difference in structure and length between the two forms (the *trans* and *cis* isomer) of this photoswitch is relatively large.[20,21]

We hypothesized that both chromone groups need to bind to the CBP for the drug to have an optimal inhibitory effect (Figure 1B). With this in mind we designed three photoswitchable inhibitors called Di-AzoChromone (DAC), *Meta*-AzoChromone (MAC) and *Para*-AzoChromone (PAC) (Fig. 1A). The first photoswitchable inhibitor, DAC, was designed in such a way that the azobenzene photoswitch intergrates two chromone groups in its structure. This reduced the length of the spacer as compared to DSCG and rendered the molecule more rigid, which could result in a larger difference in inhibitory activity between the two photoisomeric forms. DAC seemed to be a more potent mast-cell activation inhibitor than DSCG (*vide infra,* Fig. 3), however, a significant change in inhibitory activity after photoisomerization was not observed (Fig. 4). Therefore two second-generation photoswitchable inhibitors were designed. Compounds MAC and PAC (Figure 1A) consist of an azobenzene moiety with two chromone groups linked to it in the *meta* and *para* position, respectively. A methylene group was placed in-between the azobenzene and chromone groups to introduce more flexibility in the molecule. This flexibility might be favorable when two chromone groups need to interact with the CBP at the same time (Fig. 1B).

**Synthesis**

The photoswitchable inhibitor DAC was synthesized starting from chromone ethyl ester (1) (Scheme 1), which was obtained from hydroxyacetophenone and diethyl oxalate.[22] Compound **1** was converted into nitroso chromone **4** via sequential nitration,[23] reduction[23] and oxidation reactions. Azobenzene formation from precursors **3** and **4** and a sequential saponification step afforded the photoswitchable inhibitor DAC **(6).**

Compounds MAC and PAC were prepared in six steps as depicted in Scheme 2. The key features of these syntheses include a formation of a diazo compound by a reaction between *m*/*p*-toluidine and *m*/*p*-nitroso toluene **(8a**

and **8b**) to afford methylated azobenzenes **(9a** and **9b),** which were subjected to radical bromination to yield compound **10a** and **10b.** *Via* a Williamson reaction, 5-hydroxychromone methyl ester **7,** obtained from 1,2-dihydroxyacetophenone and dimethyl oxalate,[24] was introduced to both methylene groups of the azobenzenes **10a** and **10b** resulting in **11a** and **11b.** A saponification step afforded the sodium salts of the photoswitchable inhibitors MAC **(12a)** and PAC **(12b).**

Scheme 1[a]

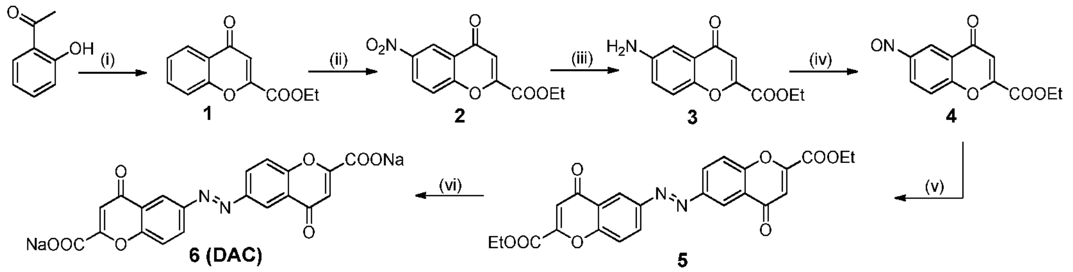

1 2 3 4 5 6 (DAC)

[a]Reagents and conditions: (i) diethyl oxalate, NaOEt/EtOH, $H_2SO_4$ (75%); (ii) $H_2SO_4$, $HNO_3$, rt, 1 h (81%); (iii) $H_2$, Pd/C, benzene, rt, 16 h (75%); (iv) Oxone, DCM/water, rt, 2 h (70%); (v) compound 3, glacial AcOH, rt, 2 d (43%); (vi) EtOH, NaOH, reflux, 2 h (91%). Scheme 2[a]

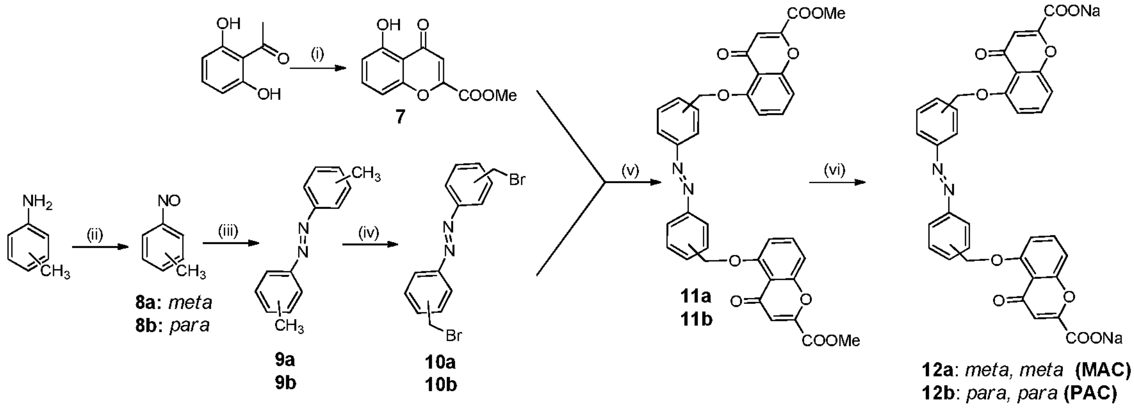

7

8a: *meta*
8b: *para*

9a
9b

10a
10b

11a
11b

12a: *meta, meta* **(MAC)**
12b: *para, para* **(PAC)**

[a]Reagents and conditions: (i) dimethyl oxalate, NaOMe/MeOH, HCl (33%); (ii) Oxone, DCM/water, rt, 1 h (**8a:** 54%; **8b**: 25%); (iii) *m*/*p*-toluidine, glacial AcOH, rt, 16 h (**9a:** 55%; **9b**: 86%); (iv) NBS, AIBN, $CCl_4$, reflux, 16 h (**10a:** 35%; **10b:** 42%); (v) $Cs_2CO_3$, MeCN, reflux, 5 h (**11a:** 70%; **11b**: 35%); (vi) EtOH, NaOH, reflux, 2 h (**12a**: 86%; **12b**: 91%).

**Photoswitchable behavior of designed mast cell activation inhibitors**

The photoswitchable behavior of DAC, MAC and PAC was studied using UV-VIS spectroscopy and RP-HPLC. The *trans*-isomers of DAC, MAC and PAC have a characteristic absorbance maximum between 300 and 400 nm in water. This absorbance maximum decreases when the molecules are photoswitched to their cis-isomers and a new absorbance maximum appears around 430 nm (Fig. 2A-C).[25] Isosbestic points at 405 nm (DAC), 270 nm (MAC) and 270 nm (PAC) clearly illustrate the selective isomerization and reversibility of the photoswitching process. Using RP-HPLC, the ratio of photoisomers was determined for both the non-irradiated and 365 nm-irradiated forms of DAC, MAC and PAC (Table 1). Higher photostationary states were obtained when the photoswitchable inhibitors were irradiated with 365 nm light in DMSO as compared to water (Table 1). Therefore, the compounds were switched at a high concentration in DMSO and diluted in water to obtain a maximum concentration of 1% DMSO (v/v). Thus obtained samples were used for inhibitory activity studies. The inhibitors could be photoisomerized by alternating irradiation at 365 nm and 400-700 nm, for at least 5 times showing little fatigue (Fig. 2D-F).

**Table 1** Ratio of *trans* and *cis* isomers of the photoswitchable inhibitors under different conditions. Ratios are determined at the isosbestic point (DAC=405 nm, MAC=270 nm, PAC=270 nm) with RP-HPLC (C18, flow 1 mL/min, MeCN and $H_3PO_4$pH=3.0).

| | DAC (*trans:cis*) | MAC (*trans:cis*) | PAC (*trans:cis*) |
|---|---|---|---|
| Non-Irradiated | 99:1 | 88:12 | 91:9 |
| Irradiated (water) | 59:41 | 45:55 | 56:44 |
| Irradiated (DMSO) | 48:52 | 38:62 | 19:81 |

**Inhibitory activity** The inhibitory activity of DAC, MAC and PAC was determined on the LAD2[26] mast cell line using a β-hexosaminidase release assay.[27] β-Hexosaminidase is an enzyme that is secreted by mast cells upon activation and therefore its activity is a reliable measure for the degree of mast cell degranulation.[28] LAD2 cells were incubated with different concentrations of DSCG and non-irradiated DAC, MAC and PAC for 30 min after which degranulation was evoked by the secretagogue Compound 48/80.[29] From Fig. 3, it is apparent that DAC, MAC and PAC show significantly greater inhibitory activity than the original drug DSCG. MAC and PAC seem to have an almost 100 times greater inhibitory potency than DSCG. Furthermore, MAC and DAC show greater efficacy than PAC.

At the concentration of Compound 48/80 (1.0 μM) that was used in this assay, DSCG did not show any inhibitory activity. At lower concentrations of Compound 48/80 (0.1 μM), DSCG was able to inhibit mast cell degranulation (data not shown).

Next, the difference in inhibitory activity between the two photoisomers of DAC, MAC and PAC was determined. The inhibitory activity was determined for each compound at two concentrations that were on the steep part of the concentration response curve, because at these concentrations a possible difference in inhibitory activity is best detectable. LAD2 cells were incubated with either non-irradiated solutions of DAC, MAC and PAC or with solutions that were irradiated with 365 nm light prior incubation. After evoking a degranulation with Compound 48/80, β-hexosaminidase release was determined.. A significant difference was observed between the irradiated and non-irradiated form of PAC. At a concentration of 10 μM the relative difference in inhibitory activity between the two forms of PAC is 25% (see Figure 4). The non-irradiated form, which consists of 91% *trans*-PAC in the photo stationary state (PSS), is more active than the irradiated form which is 81% *cis*-PAC in the PSS.

REFERENCES

(1) Sly, R. M. Ann. Allergy Asthma Immunol. 1999, 82, 233.

(2) Ghouri, N. et al. J. R. Soc. Med. 2008, 101, 466.

(3) Horak, F. Expert Opin. Investig. Drugs 2011, 20, 981.

(4) Di Capite, J. L et al.. Curr. Opin. Allergy Clin. Immunol. 2011, 11, 33.

(5) Mandhane, S. N. et al. Int. Immunopharmacol. 2011, 11, 1646.

(6) Barnes, P. J. Br. J. Pharmacol. 2006, 147, S297.

(7) McDonough, A. K. et al. Curr. Opin. Rheumatol. 2008, 20, 131.

(8) Schacke, H. et al. Pharmacol. Ther. 2002, 96, 23.

(9) Mullane, K. Biochem. Pharmacol. 2011, 82, 586.

(10) Spataro, A. C. et al. Biochem. Pharmacol. 1976, 25, 505.

(11) Abbas, A. K. et al. Immediate Hypersensitivity. In Cellular and Molecular Immunology, 6th Edition. Saunders Elsevier. 2007.

(12) Schwartz, L. B. et al. J. Invest. Dermatol. 1980, 74, 349.

(13) Metcalfe, D. D. et al. Physiol. Rev. 1997, 77, 1033.

(14) Klemm, S.; Ruland, J. Immunobiology 2006, 211, 815.

(15) Mazurek, N.; et al. P. Natl. A. Sci-Biol. 1983, 80, 6014.

(16) Mazurek, N. et al. Nature 1980, 286, 722.

(17) Foreman, J. C. et al. Brit. Med. J. 1976, 1, 820.

(18) Howell, J. B. L. et al. The Lancet 1967, 290, 539.

(19) Cairns, H. et al. J. Med. Chem. 1972, 15, 583.

(20) Beharry, A. A. et al. Chem. Soc. Rev. 2011, 40, 4422.

(21) Mendonça, C. R. B., D. T. et al. Optically Induced Processes in Azopolymers. In Molecular Switches, 2nd Edition; Feringa, B. L., Browne, W. R., Eds.; John Wiley & Sons, Ltd. 2011.

(22) Walenzyk, T.et al.Tetrahedron 2005, 61, 7366.

(23) Barker, G.; Ellis, G. P. J. Chem. Soc. C. 1970, 2230.

(24) Wu, L. et al. J. Am. Chem. Soc. 2009, 131, 7430.

(25) Bandara, H. M. D.; Burdette, S. C. Chem. Soc. Rev. 2012, 41.

(26) Kirshenbaum, A. S. et al. Leuk. Res. 2003, 27, 677.

(27) Passante, E. et al. Inflamm. Res. 2009, 58, 611.

(28) Schwartz, L. B. et al.J. Immunol. 1979, 123, 1445.

(29) Paton, W. D. M. Brit. J. Pharm. Chemoth. 1951, 6, 499.

(30) Khandwala, A. et al. Int. J. Immunopharmacol. 1983, 5, 491.

(31) Samanta, S. et al. Angew. Chem.-Int. Edit. 2012, 51, 6452.

(32) Zhu, D. et al. Org. Lett. 2008, 10, 4585.

(33) Sakamoto, R. et al. Chem. Commun. 2005.

(34) Jousselme, B. et al. Chem-Eur. J. 2003, 9, 5297.

(35) Bonardi, F. et al. Angew. Chem.-Int. Edit. 2010, 49, 7234.

## Claims

**1.** A bis-chromonyl compound of the general formula

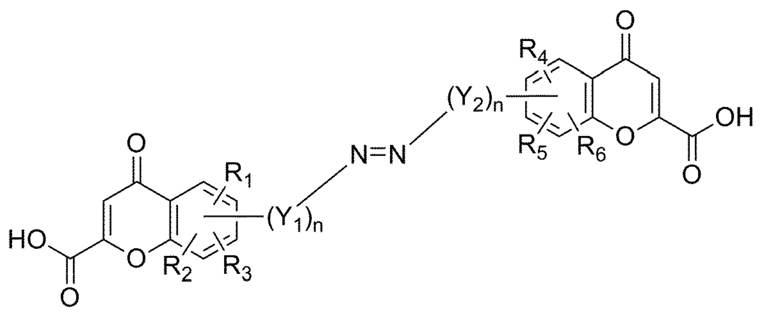

Formula I

or a pharmaceutically acceptable salt, ester or amide thereof, wherein

n is 0 or 1;
$Y_1$ is $X_1$-$Ar_1$ or O-$X_1$-$Ar_1$ and $Y_2$ is $X_2$-$Ar_2$ or O-$X_2$-$Ar_2$; wherein $Ar_1$ and $Ar_2$ are bound to N=N;

wherein $X_1$ and $X_2$ are the same or different and each is a substituted or unsubstituted straight hydrocarbon chain of 1 to 3 carbon atoms;
wherein $Ar_1$ and $Ar_2$ are the same or different and each is a 5- or 6-membered aromatic or heteroaromatic ring, optionally substituted with one or more substituents selected from the group consisting of halogen, hydroxyl, substituted or unsubstituted $C_1$-$C_4$ alkyl, substituted or unsubstituted $C_1$-$C_4$ alkoxy; and

$R_1$, $R_2$, $R_3$, $R_4$, $R_5$ and $R_6$ are the same or different and each is selected from the group consisting of H, halogen, nitro, amino, cyano, hydroxyl, substituted or unsubstituted $C_1$-$C_4$ alkyl, substituted or unsubstituted $C_1$-$C_4$ alkoxy.

**2.** Bis-chromonyl compound according to claim 1, wherein $Ar_1$ and $Ar_2$ are independently selected from the group consisting of benzene, pyridine, pyrrole, furan, thiophene, pyrazole, imidazole, isoxazole, oxazole, isothiazole, thiazole, pyridazine, pyrimidine and pyrazine.

**3.** Bis-chromonyl compound according to claim 1 or 2, wherein $Y_1$ is O-$X_1$-$Ar_1$ and/or $Y_2$ is O-$X_2$-$Ar_2$, preferably wherein $Y_1$ is O-$X_1$-$Ar_1$ and $Y_2$ is O-$X_2$-$Ar_2$.

**4.** Bis-chromonyl compound according to any of claims 1 to 3, wherein $Y_1$ and $Y_2$ are the same, preferably wherein $Y_1$ and $Y_2$ are O-X-Ar, wherein Ar is selected from the group consisting of benzene, pyridine, pyrrole, furan, thiophene, more preferably benzene.

**5.** Bis-chromonyl compound according to any of the preceding claims, wherein each of $R_2$, $R_3$, $R_5$ and $R_6$ is hydrogen, preferably wherein each of $R_1$, $R_2$, $R_3$, $R_4$, $R_5$ and $R_6$ is hydrogen.

**6.** Bis-chromonyl compound selected from

*(E)*-6,6'-(diazene-1,2-diyl)bis(4-oxo-4H-chromene-2-carboxylate) (DAC)
(*E*)-5,5'-(((diazene-1,2-diylbis(3,1-phenylene))bis(methylene)) bis(oxy))bis(4-oxo-4H-chromene-2-carboxylate) (MAC)
*(E)*-5,5'-(((diazene-1,2-diylbis(4,1-phenylene))bis(methylene))bis(oxy)) bis(4-oxo-4H-chromene-2-carboxylate)(PAC)
(*E*)-5,5'-(((5,5'-(diazene-1,2-diyl)bis(pyridine-5,3-diyl))bis(methylene))bis(oxy))bis(4-oxo-4H-chromene-2-carboxylic acid)
(*E*)-5,5'-(((4,4'-(diazene-1,2-diyl)bis(thiazole-4,2-diyl))bis(methylene))bis(oxy))bis(4-oxo-4H-chromene-2-carboxylic acid)
(*E*)-6-(2-(4-((3-(2-carboxy-4-oxo-4H-chromen-5-yl)phenyl)diazenyl)thiophen-2-yl)ethyl)-4-oxo-4H-chromene-2-carboxylic acid; and
(*E*)-6-((5-((2-carboxy-4-oxo-4H-chromen-6-yl)diazenyl)furan-2-yl)oxy)-4-oxo-4H-chromene-2-carboxylic acid

**7.** A pharmaceutical composition comprising a bis-chromonyl compound according to any of claims 1-6 and a pharmaceutically active carrier, diluent or excipient.

8. Pharmaceutical composition according to claim 7 formulated as nasal spray, inhalator, eye drops, oral formulation, nebulizer solution or topical formulation.

9. Pharmaceutical composition according to claim 8, comprising at least one further ingredient capable of inhibiting the release of mediators from mast cells.

10. A bis-chromonyl compound according to any of claims 1 to 6 for use as a therapeutic or prophylactic agent.

11. Bis-chromonyl compound according to any one of claims 1-6 for use in a method of treatment or prophylaxis of a disease involving unwanted or excessive mast cell activation, preferably wherein the disease is an allergic disease.

12. Bis-chromonyl compound for use according to claim 11, wherein the disease is selected from the group consisting of asthma, allergic rhinitis, mastocytosis, dermatographic urticaria, eczema, psoriasis, ulcerative colitis, food allergy, vulvar vestibulitis.

13. Bis-chromonyl compound according to any one of claims 1-6 for use as *in vitro* mast cell stabilizer.

14. Bis-chromonyl compound according to any one of claims 1-6 for use in a method for alleviating symptoms associated with an autoimmune disorder, preferably rheumatoid arthritis, bullous pemphigoid or multiple sclerosis, the method comprising administering to a mammalian subject in need thereof an effective dose of a bis-chromonyl compound according to any one of claims 1-7.

15. Bis-chromonyl compound according to any one of claims 1-6 for use in a method for treatment of a pathological mineral resorptive state, the method comprising administering to a subject in need thereof a bis-chromonyl compound according to any one of claims 1-6, preferably wherein said bis-chromonyl compound is administered in combination with one or more anti-osteoporotic agents, more preferably wherein said one or more anti-osteoporotic agent is selected from anabolic steroids, various phosphorus-containing agents, vitamin D and related substances, estrogenic steroids, calcitonin and aromatic carboxylic acids.

**Patentansprüche**

1. Bis-Chromonylverbindung der allgemeinen Formel

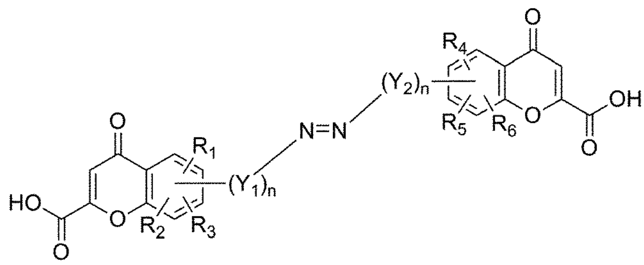

Formel I

oder ein pharmazeutisch unbedenkliches Salz, Ester oder Amid davon,
wobei

n 0 oder 1 ist;
$Y_1$ $X_1$-$Ar_1$ oder O-$X_1$-$Ar_1$ ist und $Y_2$ $X_2$-$Ar_2$ oder O-$X_2Ar_2$ ist; wobei $Ar_1$ und $Ar_2$ an N=N gebunden sind;
wobei $X_1$ und $X_2$ gleich oder verschieden sind und jedes eine substituierte oder unsubstituierte gerade Kohlenwasserstoffkette von 1 bis 3 Kohlenstoffatomen ist;
wobei $Ar_1$ und $Ar_2$ gleich oder verschieden sind und jedes ein 5- oder 6-gliedriger aromatischer oder heteroaromatischer Ring ist, optional substituiert mit einem oder mehreren Substituenten, ausgewählt aus der Gruppe bestehend aus Halogen, Hydroxyl, substituiertem oder unsubstituiertem $C_1$-$C_4$ Alkyl, substituiertem oder unsubstituiertem $C_1$-$C_4$ Alkoxy; und
$R_1$, $R_2$, $R_3$, $R_4$, $R_5$ und $R_6$ gleich oder verschieden sind und jedes ausgewählt ist aus der Gruppe bestehend aus H, Halogen, Nitro, Amino, Cyano, Hydroxyl, substituiertem oder unsubstituiertem $C_1$-$C_4$ Alkyl, substituiertem oder unsubstituiertem $C_1$-$C_4$ Alkoxy.

2. Bis-Chromonylverbindung nach Anspruch 1, wobei $Ar_1$ und $Ar_2$ unabhängig ausgewählt sind aus der Gruppe bestehend aus Benzol, Pyridin, Pyrrol, Furan, Thiophen, Pyrazol, Imidazol, Isoxazol, Oxazol, Isothiazol, Thiazol, Pyridazin, Pyrimidin und Pyrazin.

3. Bis-Chromonylverbindung nach Anspruch 1 oder 2, wobei $Y_1$ O-$X_1$-$Ar_1$ ist und/oder $Y_2$ O-$X_2$-$Ar_2$ ist, bevorzugt wobei $Y_1$ O-$X_1$-$Ar_1$ ist und $Y_2$ O-$X_2$-$Ar_2$ ist.

4. Bis-Chromonylverbindung nach einem der Ansprüche 1 bis 3, wobei $Y_1$ und $Y_2$ gleich sind, bevorzugt wobei $Y_1$ und $Y_2$ O-X-Ar sind, wobei Ar ausgewählt ist aus der Gruppe bestehend aus Benzol, Pyridin, Pyrrol, Furan, Thiophen, besonders bevorzugt Benzol.

5. Bis-Chromonylverbindung nach einem der vorhergehenden Ansprüche, wobei jedes von $R_2$, $R_3$, $R_5$ und $R_6$ Wasserstoff ist, bevorzugt wobei jedes von $R_1$, $R_2$, $R_3$, $R_4$, $R_5$ und $R_6$ Wasserstoff ist.

6. Bis-Chromonylverbindung, ausgewählt aus

   *(E)*-6,6'-(Diazen-1,2-diyl)bis(4-oxo-4H-chromen-2-carboxylat) (DAC)
   *(E)*-5,5'-(((Diazen-1,2-diylbis(3,1-phenylen))bis(methylen))bis(oxy))bis(4-oxo-4H-chromen-2-carboxylat) (MAC)
   *(E)*-5,5'-(((Diazen-1,2-diylbis(4,1-phenylen))bis(methylen))bis(oxy))bis(4-oxo-4H-chromen-2-carboxylat) (PAC)
   *(E)*-5,5'-(((5,5'-(Diazen-1,2-diyl)bis(pyridin-5,3-diyl))bis(methylen))bis(oxy))bis(4-oxo-4H-chromen-2-carboxylsäure)
   *(E)*-5,5'-(((4,4'-(Diazen-1,2-diyl)bis(thiazol-4,2-diyl))bis(methylen))bis(oxy))bis(4-oxo-4H-chromen-2-carboxylsäure)
   *(E)*-6-(2-(4-((3-(2-Carboxy-4-oxo-4H-chromen-5-yl)phenyl)diazenyl)thiophen-2-yl)ethyl)-4-oxo-4H-chromen-2-carboxylsäure; und
   *(E)*-6-((5-((2-Carboxy-4-oxo-4H-chromen-6-yl)diazenyl)furan-2-yl)oxy)-4-oxo-4H-chromen-2-carboxylsäure.

7. Pharmazeutische Zusammensetzung, umfassend eine Bis-Chromonylverbindung nach einem der Ansprüche 1-6 und einen pharmazeutisch aktiven Träger, Verdünnungsmittel oder Exzipienten.

8. Pharmazeutische Zusammensetzung nach Anspruch 7, formuliert als Nasenspray, Inhalator, Augentropfen, orale Formulierung, Zerstäuberlösung oder topische Formulierung.

9. Pharmazeutische Zusammensetzung nach Anspruch 8, umfassend mindestens einen weiteren Bestandteil, der in der Lage ist, die Freisetzung von Mediatoren aus Mastzellen zu hemmen.

10. Bis-Chromonylverbindung nach einem der Ansprüche 1 bis 6 zur Verwendung als ein therapeutisches oder prophylaktisches Agens.

11. Bis-Chromonylverbindung nach einem der Ansprüche 1-6 zur Verwendung in einem Verfahren zur Behandlung oder Vorbeugung einer Krankheit, beinhaltend unerwünschte oder übermäßige Mastzellenaktivierung, bevorzugt wobei die Krankheit eine allergische Krankheit ist.

12. Bis-Chromonylverbindung zur Verwendung nach Anspruch 11, wobei die Krankheit ausgewählt ist aus der Gruppe bestehend aus Asthma, allergischer Rhinitis, Mastocytose, dermatografischer Urtikaria, Ekzem, Psoriasis, ulzerativer Kolitis, Lebensmittelallergie, vulvärer Vestibulitis.

13. Bis-Chromonylverbindung nach einem der Ansprüche 1-6 zur Verwendung als *in vitro* Mastzellstabilisator.

14. Bis-Chromonylverbindung nach einem der Ansprüche 1-6 zur Verwendung in einem Verfahren zur Linderung von Symptomen in Zusammenhang mit einer Autoimmunstörung, bevorzugt rheumatoider Arthritis, bullösem Pemphigoid oder multipler Sklerose, das Verfahren umfassend Verabreichen einer wirksamen Dosis einer Bis-Chromonylverbindung nach einem der Ansprüche 1-7 an einen Säugerpatienten, der dies benötigt.

15. Bis-Chromonylverbindung nach einem der Ansprüche 1-6 zur Verwendung in einem Verfahren zur Behandlung eines pathologischen mineralresorptiven Zustands, das Verfahren umfassend Verabreichen einer Bis-Chromonylverbindung nach einem der Ansprüche 1-6 an einen Patienten, der dies benötigt, bevorzugt wobei die Bis-Chromonylverbindung in Kombination mit einem oder mehreren antiosteoporotischen Agenzien verabreicht wird, besonders bevorzugt wobei das eine oder die mehreren antiosteoporotische(n) Agens/ Agenzien ausgewählt ist aus anabolischen Steroiden, verschiedenen phosphorhaltigen Agenzien, Vitamin D und verwandten Substanzen, östrogenen Steroiden, Calcitonin und aromatischen Carboxylsäuren.

**Revendications**

1. Composé de bis-chromonyle de formule générale

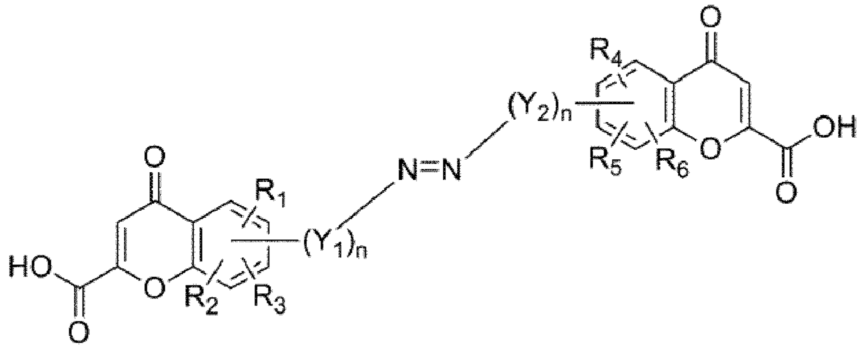

Formule I

ou un sel, ester ou amide pharmaceutiquement acceptable de celui-ci, formule dans laquelle

n vaut 0 ou 1 ;
$Y_1$ représente un groupe $X_1$-$Ar_1$ ou O-$X_1$-$Ar_1$ et $Y_2$ représente un groupe $X_2$-$Ar_2$ ou O-$X_2$-$Ar_2$ ; formules dans lesquelles $Ar_1$ et $Ar_2$ sont liés à N=N ;
dans laquelle $X_1$ et $X_2$ sont identiques ou différents et chacun est une chaîne hydrocarbonée linéaire, substituée ou non substituée de 1 à 3 atomes de carbone ;
dans laquelle $Ar_1$ et $Ar_2$ sont identiques ou différents et chacun représente un noyau aromatique ou hétéroaromatique à 5 ou 6 chaînons, facultativement substitué par un ou plusieurs substituants choisis dans le groupe constitué des atomes d'halogène et des groupes hydroxyle, alkyle en $C_1$ à $C_4$ substitué ou non substitué, alcoxy en $C_1$ à $C_4$ substitué ou non substitué ; et
$R_1$, $R_2$, $R_3$, $R_4$, $R_5$ et $R_6$ sont identiques ou différents et chacun est choisi dans le groupe constitué de l'atome d'H, des atomes d'halogène et des groupes nitro, amino, cyano, hydroxyle, alkyle en $C_1$ à $C_4$ substitué ou non substitué, alcoxy en $C_1$ à $C_4$ substitué ou non substitué.

2. Composé de bis-chromonyle selon la revendication 1, dans lequel $Ar_1$ et $Ar_2$ sont indépendamment choisis dans le groupe constitué des groupes benzène, pyridine, pyrrole, furane, thiophène, pyrazole, imidazole, isoxazole, oxazole, isothiazole, thiazole, pyridazine, pyrimidine et pyrazine.

3. Composé de bis-chromonyle selon la revendication 1 ou 2, dans lequel $Y_1$ est un groupe O-$X_1$-$Ar_1$ et/ou $Y_2$ est un groupe O-$X_2$-$Ar_2$, de préférence dans lequel $Y_1$ est un groupe O-$X_1$-$Ar_1$ et $Y_2$ est un groupe O-$X_2$-$Ar_2$.

4. Composé de bis-chromonyle selon l'une quelconque des revendications 1 à 3, dans lequel $Y_1$ et $Y_2$ sont identiques, de préférence dans lequel $Y_1$ et $Y_2$ sont un groupe O-X-Ar, formule dans laquelle Ar est choisi dans le groupe constitué des groupes benzène, pyridine, pyrrole, furane, thiophène, et est plus préférablement un groupe benzène.

5. Composé de bis-chromonyle selon l'une quelconque des revendications précédentes, dans lequel chacun de $R_2$, $R_3$, $R_5$ et $R_6$ est un atome d'hydrogène, de préférence dans lequel chacun de $R_1$, $R_2$, $R_3$, $R_4$, $R_5$ et $R_6$ est un atome d'hydrogène.

6. Composé de bis-chromonyle choisi parmi les composés suivants

(*E*)-6,6'-(diazène-1,2-diyl)bis(4-oxo-4H-chromène-2-carboxylate) (DAC)
(*E*)-5,5'-(((diazène-1,2-diylbis(3,1-phénylène))bis(méthylène))bis(oxy))bis(4-oxo-4H-chromène-2-carboxylate) (MAC)
(*E*)-5,5'-(((diazène-1,2-diylbis(4,1-phénylène))bis(méthylène))bis(oxy))bis(4-oxo-4H-chromène-2-carboxylate)(PAC)
(*E*)-5,5'-(((5,5'-(diazène-1,2-diyl)bis(pyridine-5,3-diyl))bis(méthylène))bis(oxy))bis(acide 4-oxo-4H-chromène-2-carboxylique)
(*E*)-5,5'-(((4,4'-(diazène-1,2-diyl)bis(thiazole-4,2-diyl))bis(méthylène))bis(oxy))bis(acide 4-oxo-4H-chromène-2-carboxylique) acide
(*E*)-6-(2-(4-((3-(2-carboxy-4-oxo-4H-chromén-5-yl)phényl)diazényl)thiophén-2-yl)éthyl)-4-oxo-4H-chromène-2-carboxylique ; et acide (*E*)-6-((5-((2-carboxy-4-oxo-4H-chromén-6-yl)diazényl)furan-2-yl)oxy)-4-oxo-4H-chromène-2-carboxylique

**7.** Composition pharmaceutique comprenant un composé de bis-chromonyle selon l'une quelconque des revendications 1 à 6 et un vecteur, diluant ou excipient pharmaceutiquement actif.

**8.** Composition pharmaceutique selon la revendication 7 se présentant sous la forme d'un spray nasal, d'un inhalateur, de gouttes oculaires, d'une formulation orale, d'une solution pour nébuliseur ou d'une formulation topique.

**9.** Composition pharmaceutique selon la revendication 8, comprenant au moins un autre ingrédient capable d'inhiber la libération de médiateurs à partir des mastocytes.

**10.** Composé de bis-chromonyle selon l'une quelconque des revendications 1 à 6 pour utilisation en tant qu'agent thérapeutique ou prophylactique.

**11.** Composé de bis-chromonyle selon l'une quelconque des revendications 1 à 6 pour utilisation dans un procédé de traitement ou de prophylaxie d'une maladie liée à une activation indésirable ou excessive des mastocytes, la maladie étant de préférence une maladie allergique.

**12.** Composé de bis-chromonyle pour utilisation selon la revendication 11, dans lequel la maladie est choisie dans le groupe constitué de l'asthme, de la rhinite allergique, de la mastocytose, de l'urticaire dermatographique, de l'eczéma, du psoriasis, de la rectocolite hémorragique, de l'allergie alimentaire, de la vestibulite vulvaire.

**13.** Composé de bis-chromonyle selon l'une quelconque des revendications 1 à 6 pour utilisation en tant que stabilisateur des mastocytes *in vitro.*

**14.** Composé de bis-chromonyle selon l'une quelconque des revendications 1 à 6 pour utilisation dans un procédé de soulagement des symptômes associés à un trouble auto-immun, de préférence la polyarthrite rhumatoïde, la pemphigoïde bulleuse ou la sclérose en plaques, le procédé comprenant l'administration à un sujet mammifère qui le nécessite d'une dose efficace d'un composé de bis-chromonyle selon l'une quelconque des revendications 1 à 7.

**15.** Composé de bis-chromonyle selon l'une quelconque des revendications 1 à 6 pour utilisation dans un procédé de traitement d'un état pathologique lié à la résorption des minéraux, le procédé comprenant l'administration à un sujet qui le nécessite d'un composé de bis-chromonyle selon l'une quelconque des revendications 1 à 6, de préférence dans lequel ledit composé de bis-chromonyle est administré en association avec un ou plusieurs agents anti-ostéoporose, plus préférablement dans lequel lesdits un ou plusieurs agents anti-ostéoporose sont choisis parmi les anabolisants stéroïdiens, divers agents contenant du phosphore, la vitamine D et les substances apparentées, les stéroïdes oestrogéniques, la calcitonine et les acides carboxyliques aromatiques.

Figure 1

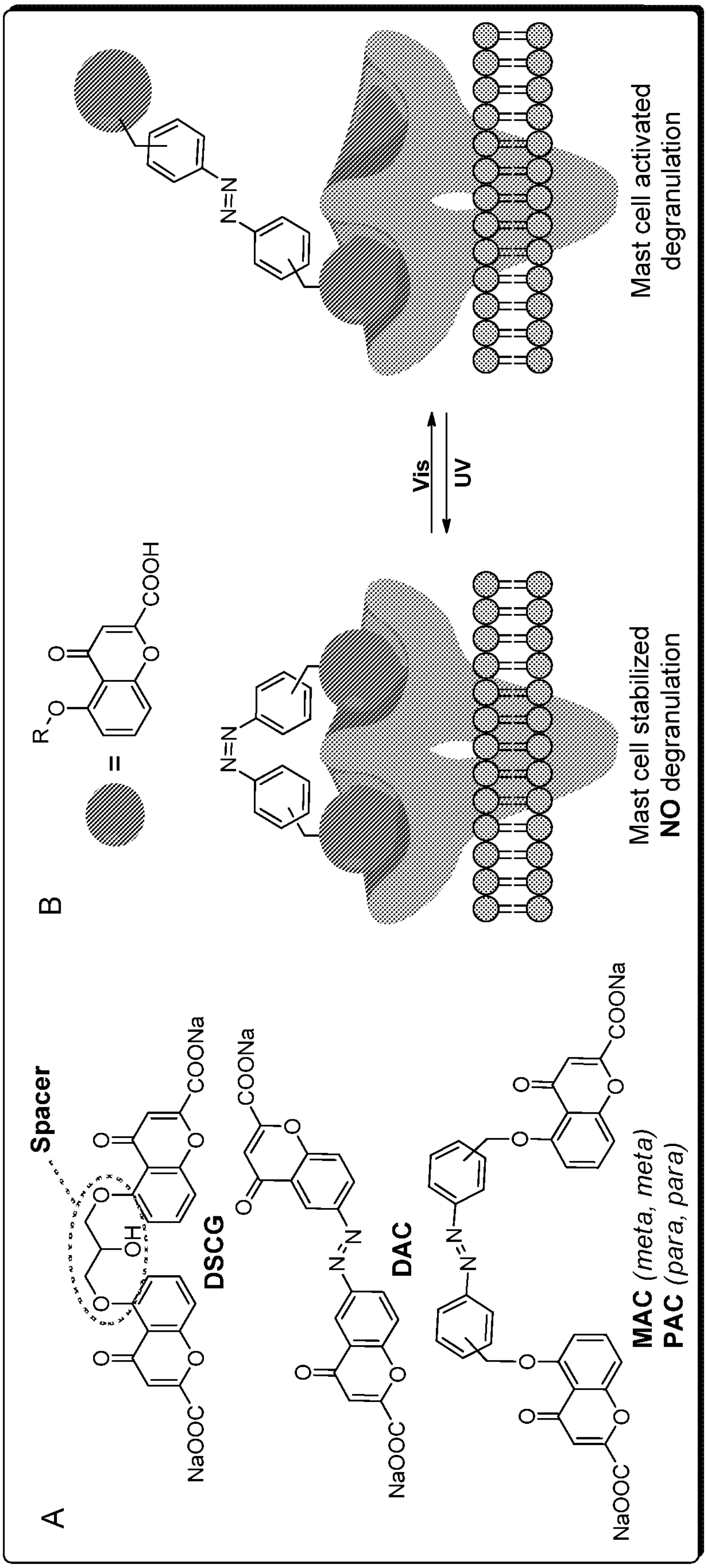
A
Spacer
DSCG
DAC
MAC (meta, meta)
PAC (para, para)
B
Vis
UV
Mast cell stabilized
NO degranulation
Mast cell activated
degranulation

Figure 2

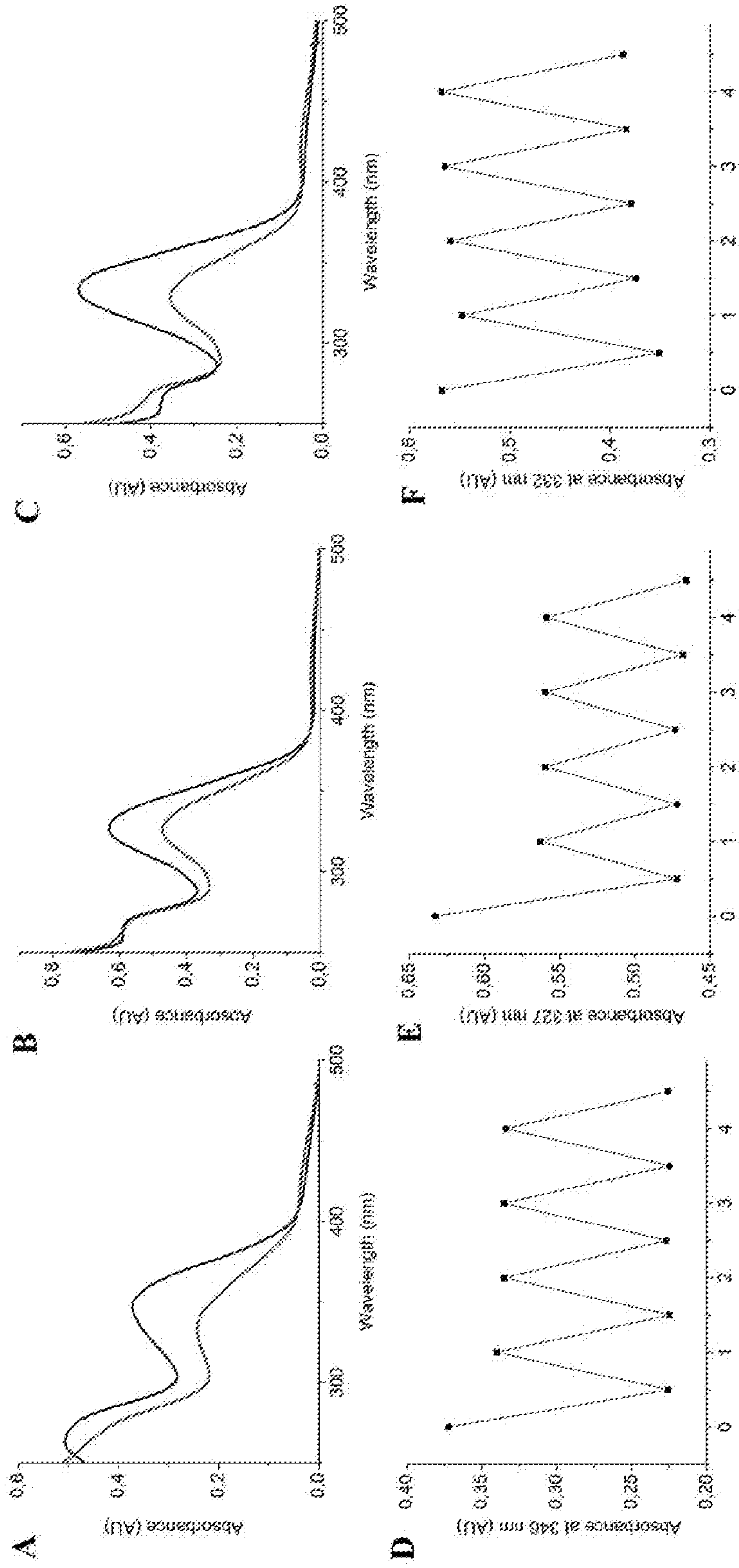

Figure 3

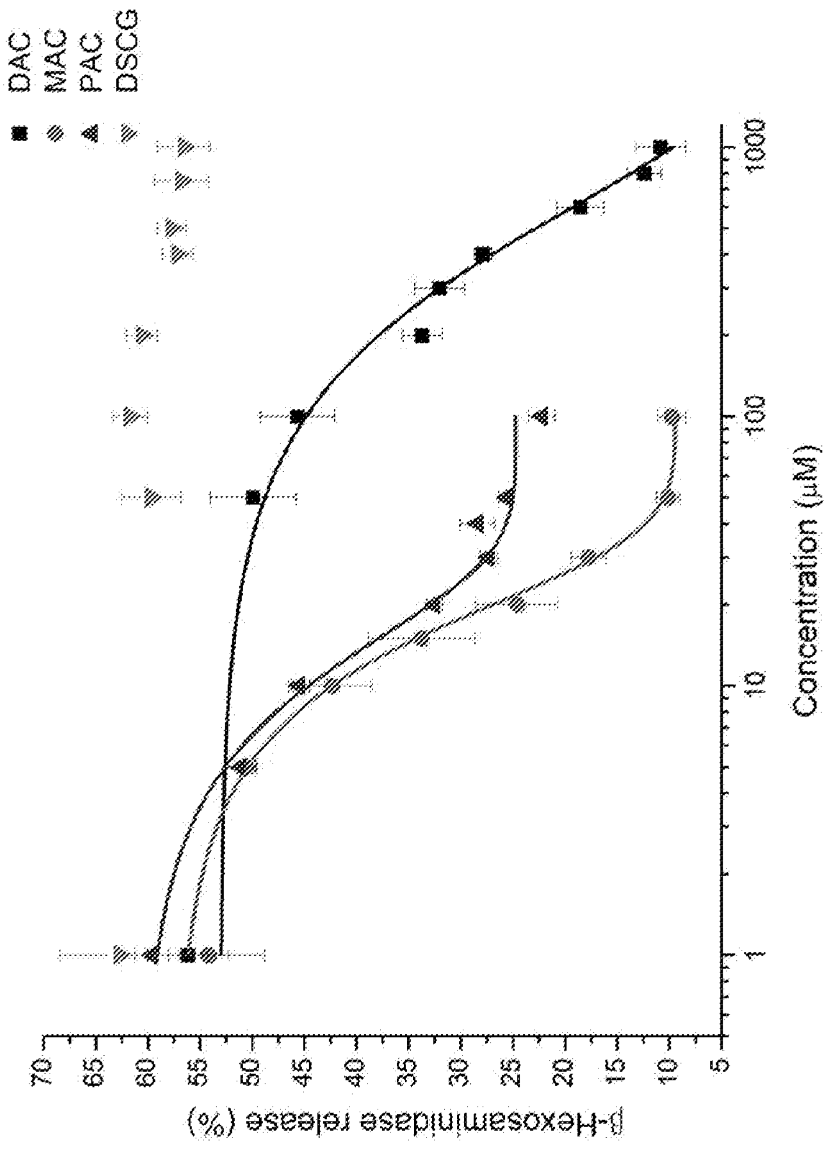
DAC
MAC
PAC
DSCG
β-Hexosaminidase release (%)
70
65
60
55
50
45
40
35
30
25
20
15
10
5
1
10
100
1000
Concentration (μM)

Figure 4

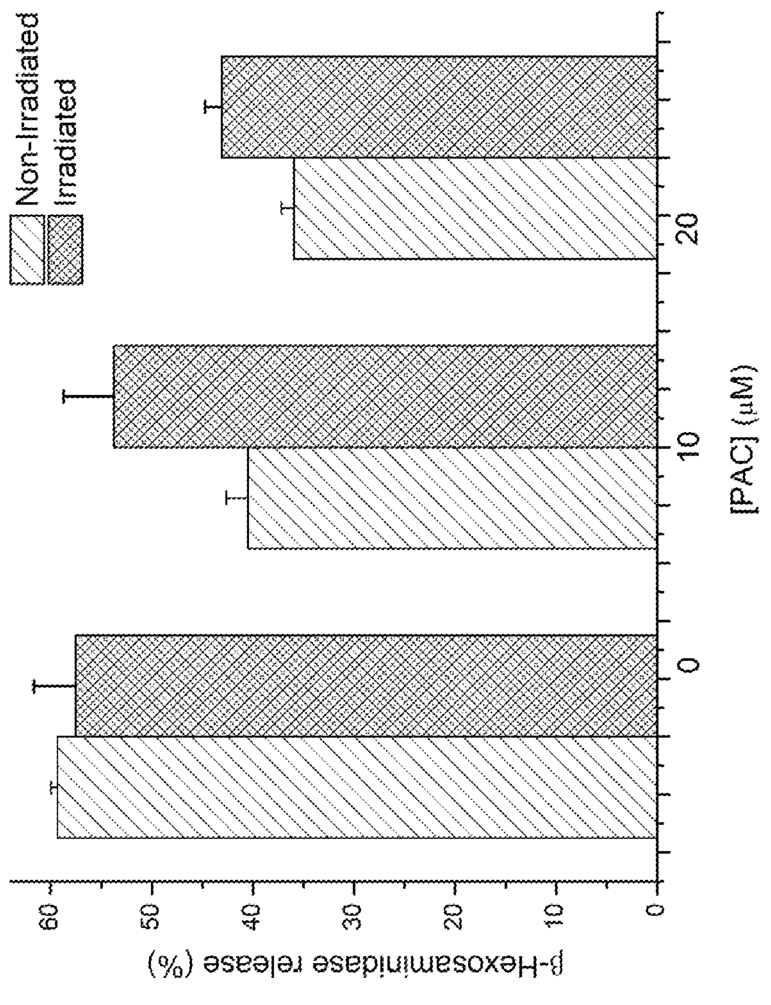
Non-Irradiated
Irradiated
β-Hexosaminidase release (%)
0
10
20
30
40
50
60
[PAC] (μM)
0
10
20

Figure 5

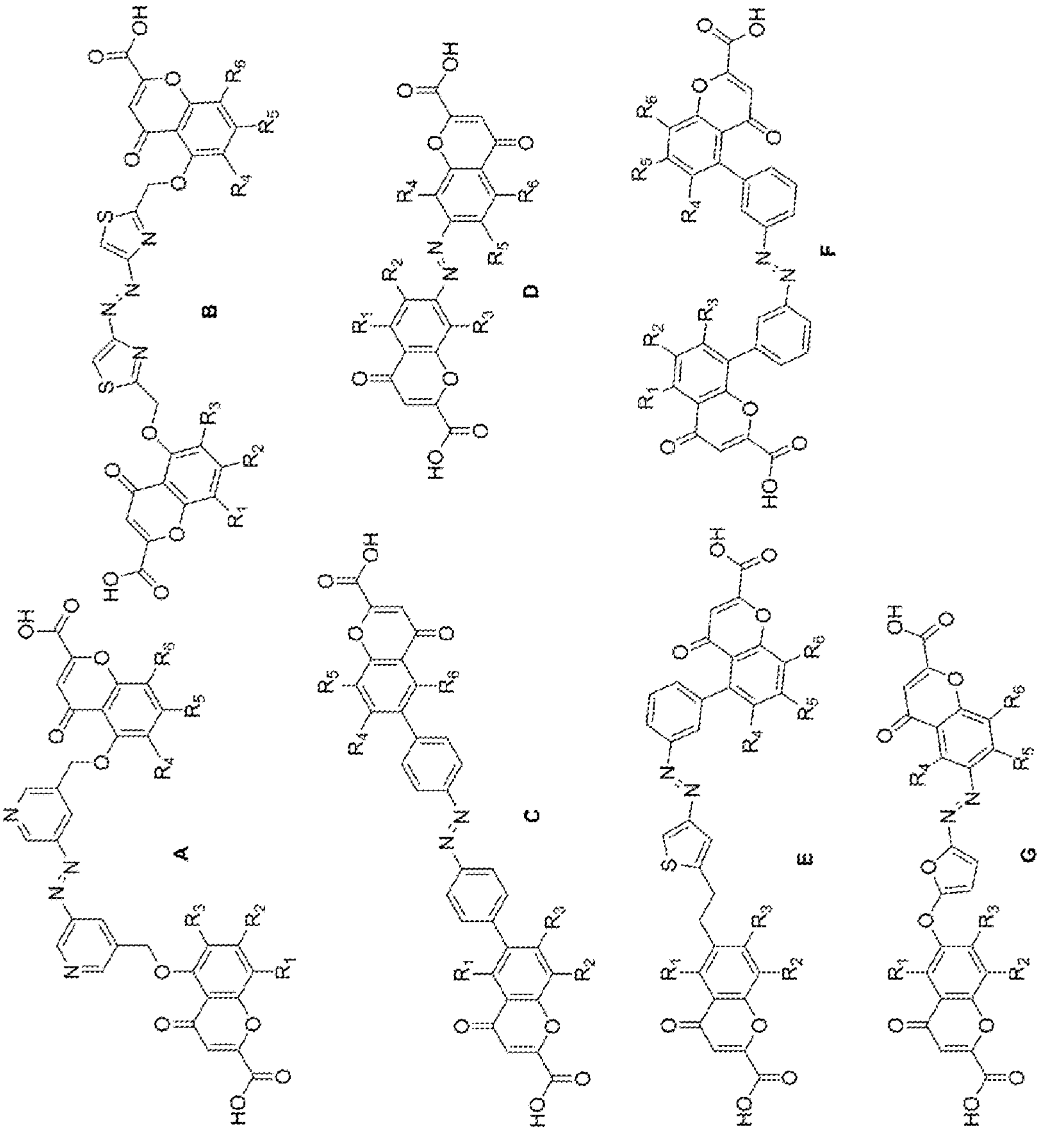

A

B

C

D

E

F

G

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3419578 A
- US 4501754 A
- US 3673218 A
- WO 2010088455 A
- US 4105781 A
- US 4125621 A
- US 4101688 A


### Non-patent literature cited in the description


- **SLY, R. M.** *Ann. Allergy Asthma Immunol.,* 1999, vol. 82, 233
- **GHOURI, N. et al.** *J. R. Soc. Med.,* 2008, vol. 101, 466
- **HORAK, F.** *Expert Opin. Investig. Drugs,* 2011, vol. 20, 981
- **DI CAPITE, J. L et al.** *Curr. Opin. Allergy Clin. Immunol.,* 2011, vol. 11, 33
- **MANDHANE, S. N. et al.** *Int. Immunopharmacol.,* 2011, vol. 11, 1646
- **BARNES, P. J.** *Br. J. Pharmacol.,* 2006, vol. 147, S297
- **MCDONOUGH, A. K. et al.** *Curr. Opin. Rheumatol.,* 2008, vol. 20, 131
- **SCHACKE, H. et al.** *Pharmacol. Ther.,* 2002, vol. 96, 23
- **MULLANE, K.** *Biochem. Pharmacol.,* 2011, vol. 82, 586
- **SPATARO, A. C. et al.** *Biochem. Pharmacol.,* 1976, vol. 25, 505
- **ABBAS, A. K. et al.** Immediate Hypersensitivity. In Cellular and Molecular Immunology. Elsevier, 2007
- **SCHWARTZ, L. B. et al.** *J. Invest. Dermatol.,* 1980, vol. 74, 349
- **METCALFE, D. D. et al.** *Physiol. Rev.,* 1997, vol. 77, 1033
- **KLEMM, S. ; RULAND, J.** *Immunobiology,* 2006, vol. 211, 815
- **MAZUREK, N. et al.** *P. Natl. A. Sci-Biol.,* 1983, vol. 80, 6014
- **MAZUREK, N. et al.** *Nature,* 1980, vol. 286, 722
- **FOREMAN, J. C. et al.** *Brit. Med. J.,* 1976, vol. 1, 820
- **HOWELL, J. B. L. et al.** *The Lancet,* 1967, vol. 290, 539
- **CAIRNS, H. et al.** *J. Med. Chem.,* 1972, vol. 15, 583
- **BEHARRY, A. A. et al.** *Chem. Soc. Rev.,* 2011, vol. 40, 4422
- Optically Induced Processes in Azopolymers. **MENDONÇA, C. R. B., D. T. et al.** Molecular Switches. John Wiley & Sons, Ltd, 2011
- **WALENZYK, T. et al.** *Tetrahedron,* 2005, vol. 61, 7366
- **BARKER, G. ; ELLIS, G. P.** *J. Chem. Soc. C.,* 1970, 2230
- **WU, L. et al.** *J. Am. Chem. Soc.,* 2009, vol. 131, 7430
- **BANDARA, H. M. D. ; BURDETTE, S. C.** *Chem. Soc. Rev.,* 2012, 41
- **KIRSHENBAUM, A. S. et al.** *Leuk. Res.,* 2003, vol. 27, 677
- **PASSANTE, E. et al.** *Inflamm. Res.,* 2009, vol. 58, 611
- **SCHWARTZ, L. B. et al.** *J. Immunol.,* 1979, vol. 123, 1445
- **PATON, W. D. M.** *Brit. J. Pharm. Chemoth.,* 1951, vol. 6, 499
- **KHANDWALA, A. et al.** *Int. J. Immunopharmacol.,* 1983, vol. 5, 491
- **SAMANTA, S. et al.** *Angew. Chem.-Int. Edit.,* 2012, vol. 51, 6452
- **ZHU, D. et al.** *Org. Lett.,* 2008, vol. 10, 4585
- **SAKAMOTO, R. et al.** *Chem. Commun.,* 2005
- **JOUSSELME, B. et al.** *Chem-Eur. J.,* 2003, vol. 9, 5297
- **BONARDI, F. et al.** *Angew. Chem.-Int. Edit.,* 2010, vol. 49, 7234